# EUROPEAN PATENT SPECIFICATION

(11) **EP 0 525 821 B1**
(45) Date of publication and mention of the grant of the patent: **29.03.2006**
(21) Application number: 92114727.8
(22) Date of filing: 26.01.1984
(51) Int. Cl.: C12Q 1/68, G01N 33/543, G01N 33/58

(54) **Methods and structures employing non-radioactive chemically-labeled polynucleotide probes**
Verfahren und Strukturen, in welchen chemisch markierte nichtradioaktive Polynukleotidesonden benutzt werden
Méthodes et structures utilisant des sondes de polynucléotides marqués chimiquement et non-radioactifs

(30) Priority: 27.01.1983 US 461469
(43) Date of publication of application: 03.02.1993
(62) Divisional of application: 84100836.0
(73) Proprietor: ENZO BIOCHEM, INC., Farmingdale, New York 11735 (US)
(72) Inventor: Stavrianopoulos, Jannis G., San Ramon, CA 94583 (US); Kirtikar, Dollie, Elmhurst, NY 11373 (US); Johnston, Kenneth H., New York, NY 10016 (US); Thalenfeld, Barbara E., New York, NY 10016 (US)
(74) Representative: Vossius & Partner

(56) References cited:
- EP-A- 0 063 879
- EP-A- 0 097 373
- WO-A-83/02277
- GB-A- 2 014 727

## Description

In the determination of the presence or the identity of genetic material, such as DNA genetic material, it has been proposed to denature the genetic material to form single-stranded DNA or single-stranded genetic material. The single-stranded genetic material is then fixed to a solid support and contacted with a probe, such as a DNA probe, having in its make up bases complementary to: the make up of the fixed genetic material to be identified and/or determined. The contacting of the single-stranded genetic material along with the single-stranded probe is carried out under conditions to effect hybridization of the genetic material to be determined or identified-and the probe.

Radioactively-labeled probes, such as radioactively-labeled single-stranded DNA probes, have been employed. U.S. Patent 4,358,535 discloses a method of identifying a pathogen present in the clinical sample by denaturing the genetic material present in the clinical sample to form single-stranded genetic material thereof and to fix the resulting single-stranded genetic material characterizing the pathogen to an inert support or surface. The thus-fixed single-stranded genetic material characterizing or identifying the pathogen is brought into contact with a radioactive single-stranded probe under hybridizing conditions to effect duplex form or double-strand formation of the genetic material derived from the pathogen and the probe. The presence of the resulting formed duplex between the probe and the pathogen genetic material would then be detected and would confirm the presence and/or identity of the pathogen.

The disadvantages of employing a radioactively-labeled probe, such as a radioactively-labeled DNA probe, for the identification of genetic material are well known to those skilled in the art. Such disadvantages include not only the precautions and hazards involved in handling the radioactive material but also the short life of such radioactive material and expense in connection with the handling and use of such radioactively-labeled DNA probes.

It is known to chemically label nucleotides and poly-nucleotides to avoid the hazards and/or difficulties associated when such compounds are radioactively-labeled. For example in the article by P.R. Langer, A.A. Waldrop and D.C. Ward entitled "Enzymatic Synthesis of Biotin-Labeled Polynucleotides: Novel Nucleic Acid Affinity Probes", in Proc. Natl. Acad. Sci., USA, Vol. 78, No. 11, pp. 6633-6637, November 1981, there are described analogs of dUTP and UTP that contain a biotin molecule bound to the. C-5 position of the pyrimidine ring through an allylamine linker arm. The biotin-labeled nueleotides are efficient substrates for a variety of DNA and RNA polymerases in vitro. Polynucleotides containing low levels of biotin substitution (50 molecules or fewer per kilobase) have denaturation, reassociation and hybridization characteristics similar to those of unsubstituted controls. Biotin-labeled polynucleotides, both single and double stranded, are selectively and quantitatively retained on avidin-Sepharose, even after extensive washing with 8M urea, 6M guanidine hydrochloride or 99% formamide. In addition, biotin-labeled nucleotides can be selectively immunoprecipitated in the presence of antibiotin antibody and Staphylococcus aurea, Protein A. These unique features of biotin-labeled polynucleotides suggest that they are useful affinity probes for the detection and isolation of specific DNA and RNA sequences.

Compounds or nucleotides have also been prepared which can be incorporated into DNA, such as double-stranded DNA, and which are useful for the preparation of non-radioactive chemically-labeled DNA probes.

In EP-A- 0 063 879 the subject matter of the above-identified article is disclosed, and additionally it is disclosed that compounds having the structure: 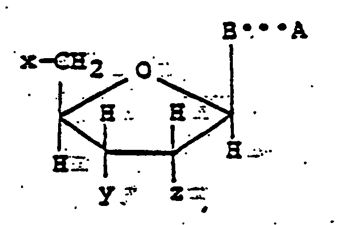 wherein B represents a purine, deazapurine, or pyrimidine moiety covalently bonded to the C^{1'}-position of the sugar moiety, provided. that when B is purine or: 7-deazapurine, it-is attached at the N⁹-position of the purine or deazapurine, and when B is pyrimidine, it is attached at the N¹-position;
wherein A represents a moiety,consisting of at least three carbon atoms which is capable of forming a detectable complex with a polypeptide when the compound is incorporated into a double-stranded ribonucleic acid, deoxyribonucleic acid duplex, or DNA-RNA hybrid;
wherein the dotted line represents a chemical linkage joining B and A, provided that if B is purine, the linkage-is attached to the 9-position of:the purine, if B is 7-deazapurine, the linkage is attached to the 7-position of the deazapurine, and if B is pyrimidine, the linkage is attached to the 5-position of the pyrimidine; and wherein each of x, y and z represents 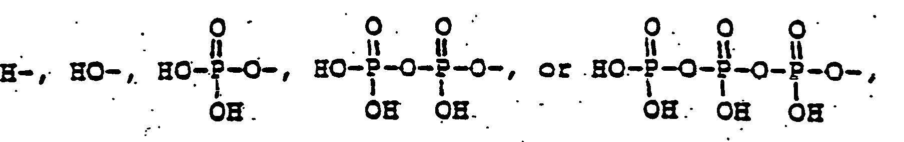 are widely useful as probes in biomedical research and recombinant DNA technology.

Particularly useful are compounds encompassed within this structure which additionally have one or more of the following characteristics: A is non-aromatic; A is at least C₅; the chemical linkage joining B and A includes an α-olefinic bond; A is biotin or iminobiotin; and B is a pyrimidine or 7-deazapurine.

EP-A-0 063 879 also discloses compounds having the structure: 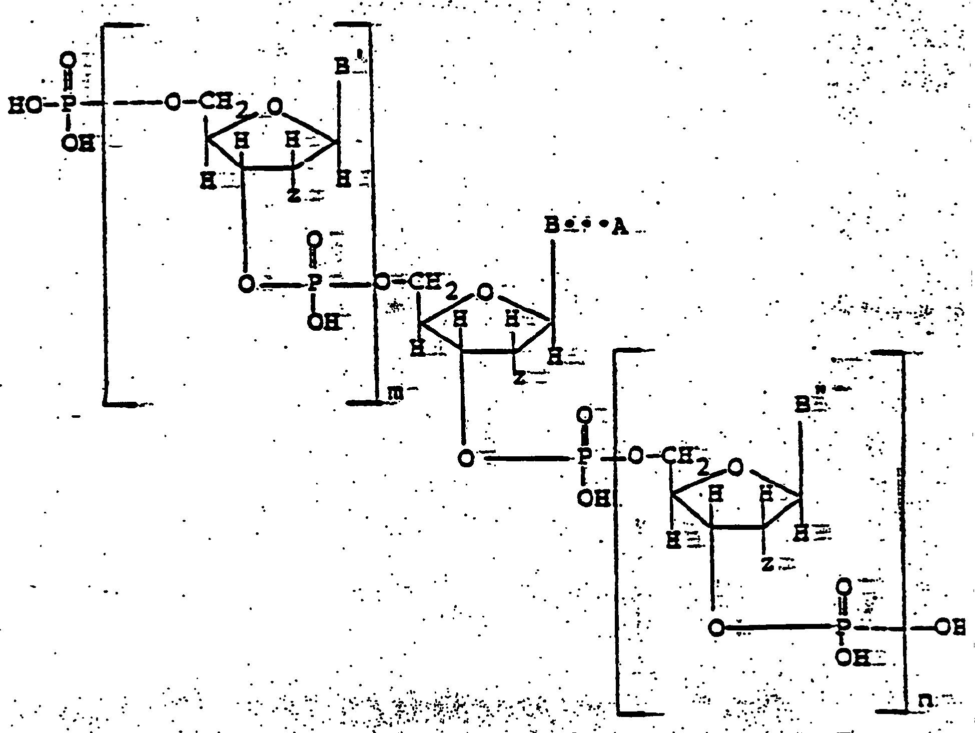 wherein each of B, B', and B" represents a purine, 7-deazapurine, or pyrimidine moiety covalently bonded to the C^{1'}-position of the sugar moiety, provided that whenever B, B', or B" is purine or 7-deazapurine, it is attached at the N⁹-position of the purine or 7-deazapurine, and whenever B, B', or B" is pyrimidine, it is attached at the N¹-position;
wherein A represents a moiety consisting of at least three carbon atoms which is capable of forming a detectable complex with a polypeptide when the compound is incorporated into a double-stranded duplex formed with a complementary ribonucleic or deoxyribonucleic acid molecule;
wherein the dotted line represents a chemical linkage joining B and A, provided that if B is purine, the linkage is attached, to the 8-position of the purine, if B is 7-deazapurine, the linkage is attached to the a-position of the deazapurine and if B is pyrimidine, the linkage is attached to the 5-position of the pyrimidine;
wherein z represents H- or HO-; and
wherein m and n represent integers from 0 up to about 100,000.

These compounds can be prepared by enzymatic polymerization of a mixture of nucleotides which include the modified nucleotides of this invention. Alternatively, nucleotides present in oligo- or polyriucleotides, may be modified using chemical methods.

The chemically-labeled or modified nucleotides described in the above-referred PNAS article and in EP-A-0 063 879 as indicated hereinabove have the structure: 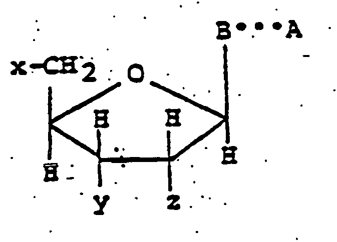 wherein B, A the dotted line and x,y and z are as defined above

Although according to EP-A-0 063 879 in principal, all compounds encompassed within the above structural formula may be prepared and used, certain of the compounds are more readily prepared or used or both, and therefore are preferred.

Thus, although according to EP-A-0 063 879 purines, pyrimidines and 7-deazapurines are in principal useful, and 7-deazapurines are preferred since purine substitution at the 8-position tends to render the nucleotides ineffective as polymerase substrates. Thus, although modified purines are useful in certain respects, they are not as generally useful as pyrimidines and 7-deazapurines. Moreover, pyrimidines and 7-deazapurines useful in that invention must not be naturally substituted at the 5- or 7- positions, respectively. As a result, certain bases, such as thymine, 5-methylcytosine, and 5-hydroxymethylcytosine, are not useful. Preferred bases are cytosine, uracil, deazaadenine and deazaguanine.

A may be any moiety which has at least three carbon atoms and is capable of forming a detectable complex with a polypeptide when the modified nucleotide is incorporated into a double-stranded duplex containing either deoxy-ribonucleic or ribonucleic acid.

A therefore may be any ligand which possesses these properties, including haptens which are only immunogenic when attached to a suitable carrier, but are capable of interacting with appropriate antibodies to produce complexes. Examples of moieties which are useful according to EP-A-0 063 879 include: 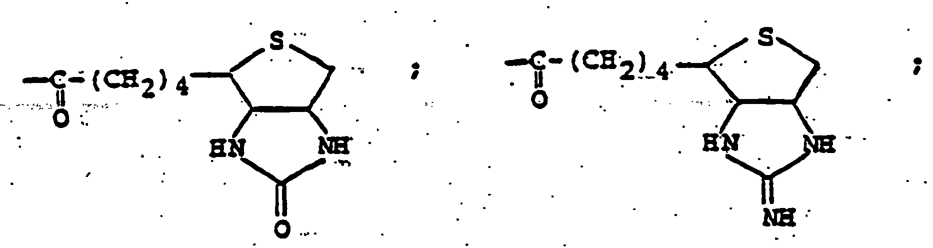 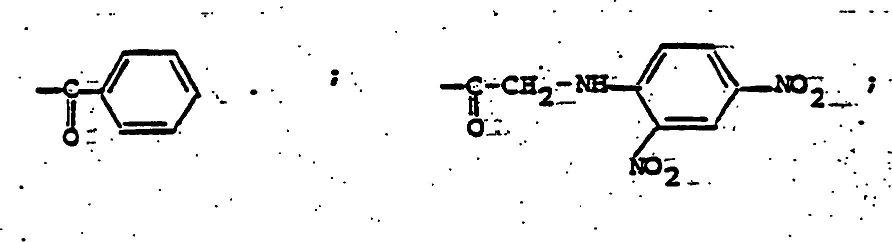 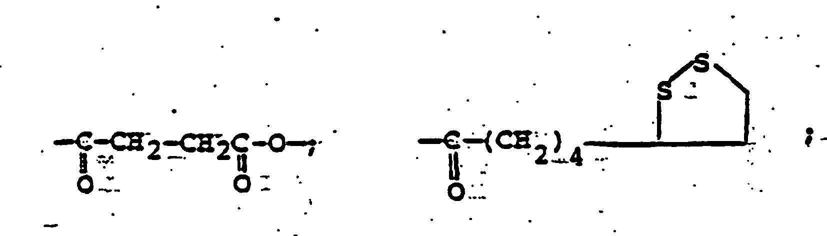 and

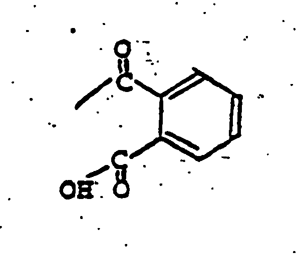

Of these, the preferred A moieties are biotin and iminobiotin.

Mcreover, since aromatic moieties tend to intercalate into a base-paired helical structure, it is preferred in EP-A-0 063 879 that the moiety A be nonaromatic. Also, since smaller moieties may not permit sufficient molecular interaction with polypeptides, it is preferred that A be at least C₅ so that sufficient interaction can occur to permit formation of stable complexes. Biotin and iminobiotin are said to satisfy both of these criteria.

According to EP-A-0 063 879 the linkage or group joining moiety A to base B may include any of the well known bonds, including carbon-carbon single bonds, carbon-carbon double bonds, carbon-nitrogen single bonds, or carbon-oxygen single bonds. However, it is generally preferred that the chemical linkage include an olefinic bond at the α-position relative to B. The presence of such an- α-olefinic bond serves to hold the moiety A away from the base when the base is paired with another in the well known double-helix configuration. This permits interaction with polypeptide to occur more readily, thereby facilitating complex formation. Moreover, single bonds with greater rotational freedom may not always hold the moiety sufficiently apart from the helix to permit recognition by and complex formation with polypeptide.

According to EP-A-0 063 879 it is even more preferred that the chemical linkage group be derived from a primary amine, and have the structure -CH₂-NH-, since such linkages are easily formed utilizing any of the well known amine modification reactions. Examples of preferred linkages derived from allylamine and allyl-(3-amino-2-hydroxy-1-propyl) ether groups have the formulae -CH=CH-CH₂-NH- and 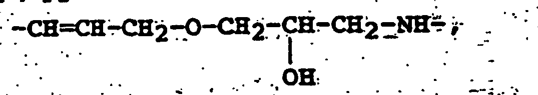 respectively.

Although these linkages are preferred, others can be used, including particularly olefin linkage arms with other modifiable functionalities such as thiol, carboxylid acid, and epoxide functionalities.

The linkage groups are attached at specific positions, namely, the 5-position of a pyrimidine, the 8-position of a purine, or the 7-position of a deazapurine. Substitution at the 8-position of a purine does not produce a modified nucleotide which is useful-in all the methods discussed in EP-A-0 063 879. It may be that the 7-position of a purine, which is occupied by a nitrogen atom, could be the point of linkage attachment. However, the chemical substitution methods employed up to that time were not suitable for this purpose.

The letters x, y, and z represent groups attached to the 5', 3' and 2' positions of the sugar moiety. They may be any of

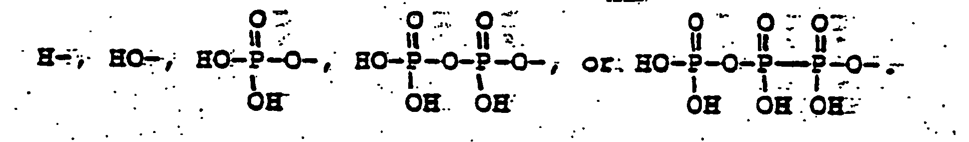

Although conceivable, it is unlikely that all of x, y, and z will simultaneously be the same. More likely, at least one of x, y, and z will be a phosphate-containing group, either mono-, di-, or tri-phosphate, and at least one will be HO- or H-. As will be readily appreciated, the most likely identity of z will be HO- or H-indicating ribonucleotide or deoxyribonucleotide, respectively. Examples of such nucleotides include 5'-ribonucleoside monophosphates, 5'-ribonucleoside diphosphates, 5'-deoxyribonucleoside triphosphates, 5'p-ribonucleoside-3'p, and 5'p-deoxyribonucleoside-3'p. More specific examples include modified nucleotides of this type in which A is biotin or iminobiotin, the chemical linkage is 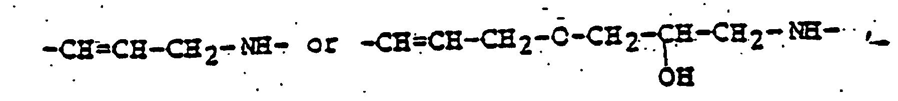 and B is uracil or cytosine.

The general synthetic approach adopted for introducing the linker arm and probe moiety onto the base is discussed thereinabove. (See especially, J.L. Ruth and D.E. Bergstrom, and M.K. Ogawa, J. Amer. Chem. Soc. 100, 8106, 1978; and C.F. Bigge, P. Kalaritis, J.R. Deck and M.P. Mertes, J. Amer. Chem. Soc. 102, 2033, 1980.) Bowever, the olef in substituents employed herein have not been used previously. To facilitate attachment of probe moiety A, it is stated in EP-A-0 063 879 as being particularly desirable to employ olefins with primary amine functional groups, such as allylamine [AA] or allyl-(3-amino-2-hydroxy-1-propyl) ether [NAGE], which permit probe attachment by standard amine modification reactions, such as, 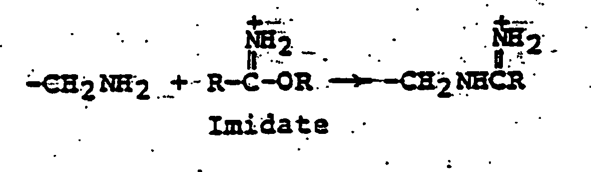 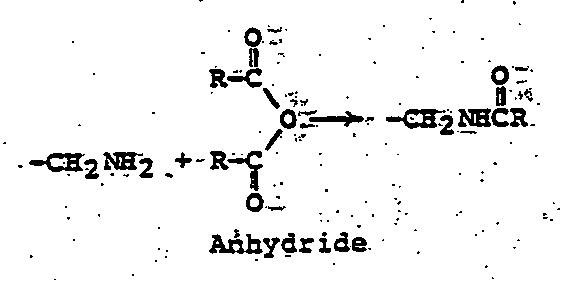 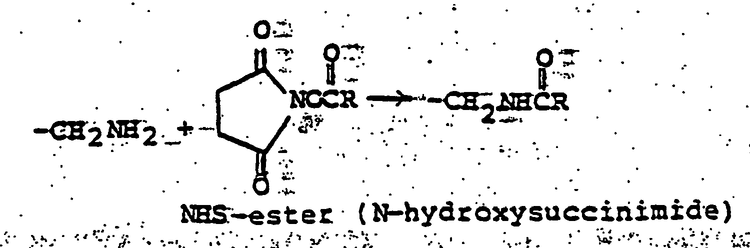 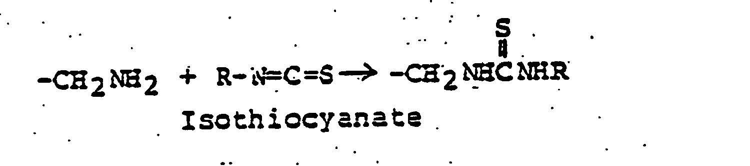 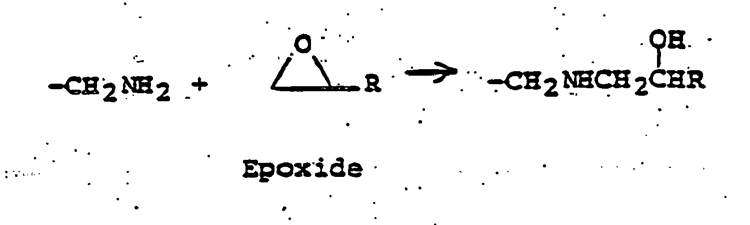 Because of ease of preparation, it is preferable according to EP-A-0 063 879 to use NHS-esters for probe addition. However, olefin linker arms with other modifiable functional groups, such as thiols, carboxylic, acids, epoxides, and the like, can also be employed. Furthermore, both linker arm and probe can be added in a single-step if deemed desirable.

Having the biotin probe directly attached to the nucleotide derivatives that are capable of functioning as enzyme substrates offers considerable versatility, both in the experimental protocols that can be performed and in the detection methods (microscopic and non-microscopic) that can be utilized for analysis. For example, biotin nucleotides can be introduced into polynucleotides which are in the process of being synthesized by cells or crude cell extracts, thus making it possible to detect and/or isolate nascent (growing) polynucleotide chains. Such a procedure is impossible to do by any direct chemical modification method. Furthermore, enzymes can be used as reagents for introducing probes such as biotin into highly selective or site-specific locations in polynuclectides; the chemical synthesis of similar probe-modified products would bes extremely difficult to achieve at best.

Further, there are disclosed in EP-A-0 097 373 modified non-radioactive chemically-labeled nucleotides wherein the nucleotides modified such as at the 5-position of pyrimidine or the 7-position of purine, preparatory for the preparation of nucleotide probes therefrom suitable for attachment to or incorporation into DNA or other nucleic acid materials. In the preparation of such modified nucleotides, the nucleotides, i.e. nucleic-acids, preferably are modified in a non-disruptive manner such that the resulting modified nucleotides are capable of incorporation into nucleic acids and once incorporated in nucleic acids, the modified nucleotides, do not significantly interfere with the formation or stabilization of the double helix formed of the resulting nucleic acids containing the modified nucleotides. The non-disruptive modification of nucleotides and nucleic acids incorporating such modified nucleotides is-in contrast with those modifications of nucleotides which are characterized as a disruptive modification in the sense that the resulting disruptively modified nucleotides and nucleic acids containing the same block proper double helix formation. In the practices of EP-A-0 097 373, the nucleotides are desirably modified at the 5-position of the pyrimidine or the 7-position of the purine. The nucleotides so modified are non-disruptively modified and nucleic acids containing such nucleotides are capable of forming a double helix arrangement.

Broadly, in another aspect of the practices of EP-A-0 097 373 , various methods are useful for the tagging or labeling of DNA in a non-disruptive manner. For example, biotin is added on the end of a DNA or RNA molecule. The addition of biotin is accomplished by addition of a ribonucleotide. The 3', 4' vicinal hydroxyl groups are oxidized by periodate oxidation and then reduced by a borohydride in the presence of biotin hydrazide. Alternatively, carbodiimide can also be used to couple biotin to the aldehyde group.

Another technique for tagging nucleic acid material such as DNA or RNA involves the addition of a large marker to the end of a DNA or RNA molecule. One example of this technique is the addition of a molecule, e.g. lysylglycine, where the amino groups are tagged with biotin. Another example would be to follow the procedure set forth hereinabove but employing carbodiimide as the cross-linking agent. Still another example of this technique would be to produce a biotinylated dA:dU double helical polymer and to ligate this polymer to the probe prepared in accordance with EP-A-0 097 373.

Another technique for tagging DNA in a non-disruptive manner involves the isolation of dPyrTP having a putricine or spermidine on the 5-position from PS16 or phage-infected cells. If desired, dPyrTP is made from phage DNA and phosphorylated to dPyrTP followed by modification of the polyamine side chain by means of standard nucleophilic reagent NHS-biotin.

Another technique for tagging DNA in a non-disruptive manner involves the addition of glucose to 5-hydroxymethylcytosine (5 HMC) in DNA using T4 phage glycosylating enzymes followed by screening by means of a lectin-based assay.

Still another method for tagging DNA in a non-disruptive manner involves 5-HMC-triphosphate made from the hydrolysis of T4-DNA followed by phosphorylation of the 5HMCMP to 5 MMCTP. 5 HMCTP is then incorporated into DNA using polymerase I. Thus, any DNA can be modified to have non-disruptively incorporated therein 5 HMC.

A method for tagging DNA in a mildly disruptive manner according to EP-A-007373 involves reacting nucleic acids in the double helical, form with alkylating reagents as for example benz(o)pyrene diol epoxide or aflatoxin. Under appropriate conditions of the N² group of guanine, the N⁴ group of adenosine or the N⁴ group of cytosine are alkylated. These modified nucleotides can be used as linking arms for the addition of a reporter molecule such as biotin.

These specially modified nucleotides suitable as non-radioactive chemical labels for DNA probes or DNA material as described in EP-A- 0.097 373 can be broadly characterized and described as phosphoric acid P moiety, a sugar or monosaccharide S moiety, a base B moiety, a purine or a pyrimidine and a signalling chemical moiety Sig covalently attached thereto, either to the P, S or B moiety.

Of special interest are those nucleotides having a general formula.

P - S - B - Sig

wherein P is the phosphoric acid moiety including mono-, di-, tri-, or tetraphosphate, S' the sugar or monosaccharide moiety, B the ..base moiety, either a purine or a pyrimidine. The phosphoric acid moiety P is attached at the 3' and/or the 5' position of the S moiety when the nucleotide is a deoxyribonucleotide and at the 2' , 3' and/or 5' position when the nucleotide is a ribonucleotide. The base B moiety is attached from the N1 position of the N9 position to the 1' position of the S moiety when the base moiety is a pyrimidine or a purine, respectively. The Sig moiety is covalently attached to the B moiety of the nucleotide and when so attached is capable of signalling itself or makes itself self-detecting or its presence known and desirably or preferably permits the incorporation of the resulting nucleotide P - S - B - Sig into or to form a double-stranded helical DNA or RNA or DNA-RNA hybrid and/or to be detectable thereon. Another special nucleotide in accordance with EP-A-0 097 373 is characterized by the general formula:

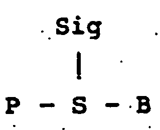

Such nucleotides would be characterized as ribonucleotides. The phosphoric acid moiety is attached at the 2', 3' and/or 5' position of the sugar S moiety and the base B being attached from the N1 position or the N9 position to the 1'- position of the sugar S moiety when said base is a pyrimidine or a purine, respectively. The Sig chemical moiety is covalently attached to the sugar S moiety is capable of signalling itself or making itself self-detecting or its presence known and preferably permits the incorporation of the ribonucleotide into its corresponding double-stranded RNA or a DNA-RNA hybrid.

Such nucleotides 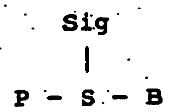 desirably have the Sig chemical moiety attached to the C2' position of the S moiety or the C3' position of the S moiety.

Still further, nucleotides in accordance with the practices of EP-A-0 097 373 include the nucleotides having the formula 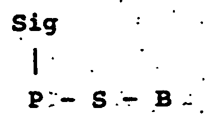 wherein P is the phosphoric acid moiety, S the sugar moiety and B the base moiety. In these special nucleotides, the P moiety is attached to the 3' and/or the position of the S moiety when the nucleotide is deoxyribonucleotide and at the 2', 3' and/or 5' position when the nucleotide is a ribonucleotide. The base B is either a purine or a pyrimidine and the B moiety is attached from the N1 or the N9 position to the 1' position of the sugar moiety when said B moiety is a pyrimidine or a purine, respectively. The Sig chemical moiety is covalently attached to the phosphoric acid P moiety via the chemical linkage 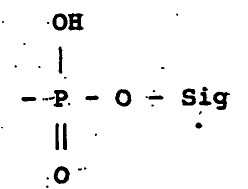 said Sig, when attached to said P moiety being capable of signalling itself or making itself self-detecting or its presence known and desirably the nucleotide is capable of being incorporated into a double-stranded polynucleotide, such as DNA, RNA or DNA-RNA hybrid and when so incorporated therein is still self-detecting.

It is pointed out that the special nucleotides in accordance with the practices of EP-A-0 097 373 described or defined hereinabove by the general formula P - S - B - Sig, also include nucleotides wherein the Sig chemical moiety is covalently attached to the B moiety at the N⁶ or 6-amino group position when the B moiety is adenine or the N² or 2-amino group position when the B moiety is guanine or the N⁴ or 4-amino group position when the B moiety is cytosine. The resulting nucleotides containing the Sig moiety attached thereto are capable of signalling themselves or making themselves self-detecting or their presence known and being detectable is a double-stranded or DNA, RNA or DNA-RNA hybrid.

By way of summary, as indicated hereinabove with respect to the make up of the various special nucleotides in accordance with EP-A-0 097 373, the special nucleotides can be described as comprising a phosphoric acid moiety P, a sugar moiety S and a base moiety B, a purine or pyrimidine, which combination of P-S-B is well known with respect to and defines nucleotides, both deoxyribonucleotides and ribonucieotides. The nucleotides are then modified by having covaldntly attached thereto, to the P moiety and/or the S moiety and/or- the B moiety, a chemical moiety Sig. The chemical moiety Sig so attached to the nucleotide P-S-B is capable of rendering or making the resulting nucleotide, now comprising P-S-B with the Sig moiety being attached to one or more of the other moieties, self-detecting or signalling itself on capable of making its presence known per se, when incorporated into a poly-nucleotide, especially a double-stranded polynucleotide, such as a double-stranded DNA, a double-stranded RNA or a double-stranded DNA-RNA hybrid. The Sig moiety desirably should not interfere with the capability of the nucleotide to form a double-stranded polynucleotide containing the special Sig-containing nucleotide in accordance with this invention and, when sorincorporated therein, the Sig-containing nucleotide is capable of detection, localization or observation.

The Sig moiety employed in the make up of the special nucleotides of EP-A-0 097 373 could comprise an enzyme or enzymatic material, such as alkaline phosphatase, glucose oxidase, horseradish peroxidase or ribonuclease. The Sig moiety could also contain a fluorescing component, such as fluorescein or rhodamine or dansyl. If desired, the Sig moiety could include a magnetic component associated or attached thereto, such as a magnetic oxide or magnetic iron oxide, which would make the nucleotide or polynucleotide containing such a magnetic-containing Sig moiety detectable by magnetic means. The Sig moiety might also include an electron dense component, such as ferritin, so as to be available by observation. The Sig moiety could also include a radioactive isotope component, such as radioactive cobalt, making the resulting nucleotide observable by radiation detecting means. The Sig moiety could also include a hapten component or per se be capable of complexing with an antibody specific thereto. Most usefully, the Sig moiety is a polysaccharide or oligosaccharide or monosaccharide, which is capable of complexing with or being attached to a sugar or polysaccharide binding protein, such as a lectin, e.g. Concanavilin A. The Sig component or moiety of the special nucleotides could also include a chemiluminescent component.

As indicated in accordance with the practices of EP-A-0 097 373, the Sig component could comprise any chemical moiety which is attachable either directly or through a chemical linkage or linker arm to the nucleotide, such as to the base B component therein, or the sugar S component therein, or the phosphoric acid P component thereof.

The Sig component of the nucleotides in accordance with EP-A-0 097 373 and the nucleotides and polynucleotides incorporating the nucleotides containing the Sig component are equivalent to and useful for the same purposes as the nucleotides described in EP-A-0 063 879. More specifically, the chemical moiety A described in EP-A-0 063 879 is functionally the equivalent of the Sig component or chemical moiety of the special. nucleotides of EP-A-0 097 373. Accordingly, the Sig component or chemical moiety of nucleotides of EP-A-0 097 373 can be directly covalently attached to the P, S or B moieties or attached thereto via a chemical linkage or linkage arm as described in EP-A-0 097 373, as indicated by the dotted line connecting B and A of the nucleotides of EP-A-0 063 879 The various linker arms or linkages identified in EB-A-0 063 879 applicable to and useful in the preparation of the special nucleotides of EP-A-0 097 373.

A particularly important and useful aspect of the special nucleotides of EP-A-0 097 373 is the use of such nucleotides in the preparation of DNA or RNA probes. Such probes would contain a nucleotide sequence substantially matching the DNA or RNA sequence of genetic material to be located and/or identified. The probe would contain one or more of the special nucleotides of this invention. A probe having a desired nucleotide sequence, such as a single-stranded polynucleotide, either DNA or RNA probe, would then be brought into contact with DNA or RNA genetic material to be identified. Upon the localization of the probe and the formation of a double-stranded polynucleotide containing the probe and the matching DNA or, RNA material to be identified, the resulting formed double-stranded DNA or RNA-containing material would then be observable and identified. A probe in accordance with EP-A-0 097 373 may contain substantially any number of nucleotide units, from about-5 nucleotides up to about 500_{'} or more, as may be required. It would appear that 12 matching, preferably consecutive, nucleotide units would be sufficient to effect an identification of most of the DNA or RNA material to be investigated or identified, if the 12 nucleotide sequence of the probe matches a corresponding cooperative sequence in the DNA or RNA material being investigated or to be identified. As indicated, such probes may contain one or more of the special Sig-containing nucleotides preferably at least about one special nucleotide per 5-10 of the nucleotides in the probe.

It in an object of the present invention to provide a method for detecting a polynucleotide sequence which comprises:
(a) fixing or immobilizing said polynucleotide sequence to a glass or plastic substrate such that the polynucleotide sequence is in a single-stranded form and is capable of hybridizing to complementary nucleic acid sequences;
(b) forming a duplex comprising said single-stranded polynucleotide sequence hybridized to an oligo- or polynucleotide probe, said probe having attached thereto a non-radioactive chemical label comprising a signalling moiety capable of generating a quantifiable signal detectable by means selected from photometric techniques, spectrophotometric techniques, visually, cotorimetric techniques, chemiluminescent techniques, fluorometric techniques and immunofluorometric techniques;
(c) generating said quantifiable signal, and
(d) detecting said quantifiable signal thereby detecting said polynucleotide sequence.

In accordance with the practice of this invention, non-radioactive chemically labeled.polynucleotides are prepared such as non-radioactive chemically labeled single-stranded DNA probes. Such probes are then brought into contact with genetic material to be identified or otherwise detected. Before these probes are brought into contact with the genetic material to be identified or investigated, the genetic material is denatured to produce or form single-stranded genetic material, therefrom, such as single-stranded DNA derived from the genetic material. The resulting single-stranded genetic material is fixed to a suitable inert support or surface which is a plastic material, e.g. polystyrene, or a transparent or translucent glass surface. Thereupon, the special non-radioactive chemically-labeled probes, such as the special non-radioactive chemically-labeled single-stranded DNA probes used in this invention are brought into contact with the thus-fixed single-stranded genetic material under hybridizing conditions. The probe is selected to provide sufficient number, e.g. at least about 25 bases, in its make up, complementary to the bases making up the genetic material to be detected or identified. The hybridization of the probe to the single stranded genetic material to be identified with resulting double-stranded or duplex formation would then be detected by means of the non-radioactive chemical label attached to the probe portion of the resulting formed double stranded hybrid or duplex hybrid. Various techniques, depending upon the non-radioactive chemical label employed in the make up of the probe, may be employed to detect the formation of the double strand or duplex hybrid. It is preferred, however, in the practices of this invention, to employ spectrophotometric techniques and/or enzyme linked immunosorbent assay (ELISA) techniques for the determination of the formed hybrid. Spectrophotometric and ELISA techniques permit not only the detection of the resulting formed double stranded hybrid, but also permit the quantitative determination thereof, such as by the enzymatic generation of a product that can be measured colorimetrically or fluorometrically. In colorimetric determination, an enzyme linked antibody, such as alkaline phosphatase linked antibody, would be attached to the non-radioactive chemically-labeled probe and employed for the enzymatic generation of the product that can be measured calorimetrically or spectrophotometrically. Another suitable spectrophotometric or colorimetric technique involves immunofluorescence, wherein fluorescein-labeled antibody is employed in the make up prior to or is attached to the non-radioactive chemical label after hybrid formation and the fluorescein-labeled antibody attached or fixed to the probe measured fluorometrically.

Spectrophotometric or colorimetric techniques, in addition to providing the above-identified quantitative result, also-usefully provide a fairly prompt visual manifestation or elicitation of the non-radioactive chemical label in the resulting formed double-stranded hybrid. Other ELISA-like or related techniques are also useful for the detection of the non-radioactive chemical label in the formed duplex, such other techniques would also involve the use of enzymes, e.g. immunoperoxidase, or the use of electron dense markers, e.g. immunoferritin, or other chemical and/or physical markers, attachable or attached to the probes. Broadly, the practices of this invention provide techniques comparable to enzyme linked immunosorbent assay techniques, not only for the qualitative, but also the quantitative determination of hybrid formation.

As indicated, it is preferred to use ELISA techniques to effect or bring about the expression or indication of, qualitatively or quantitatively, of the non-radioactive chemically-labeled probe in the resulting formed double-stranded or hybridized material, such as the duplex or hybrid formed by the non-radioactive chemically-labeled DNA probe and its substantially complementary genetic DNA material derived from the denatured genetic DNA material to be identified. In the preferred ELISA technique in accordance with this invention, single-stranded genetic material, e.g. DNA., after denaturing of the DNA material to be identified to form the single-stranded DNA material, is fixed to a substrate. In the practice of this invention it is preferred that the substrate be a transparent substrate, such a glass substrate or surface.

By employing a transparent substrate, such as a glass substrate, to which the single-stranded DNA material is fixed and hybridized, the ELISA technique provides for qualitative and quantitative determination of the DNA material to be identified. By employing a transparent substrate to which the DNA material is fixed, full realization of the benefits and versatility of the ELISA techniques applied to the practices of this invention are obtainable.

Preferred embodiments of the present invention are explained in detail in the following.

The present invention provides a method for detecting a polynucleotide sequence which comprises:
(a) fixing or immobilizing said polynucleotide sequence to a glass or plastic substrate such that the polynucleotide sequence is in a single-stranded form and is capable of hybridizing to complementary nucleic acid sequences;
(b) forming a duplex comprising said single-stranded polynucleotide sequence hybridized to an oligo- or polynucleotide probe, said probe having attached thereto a non-radioactive chemical label comprising a signalling .moiety capable of generating a quantifiable signal detectable by means selected from photometric techniques, spectrophotometric techniques, visually, colorimetric techniques, chemiluminescent techniques, fluorometric techniques and immunofluorometic-techniques;
(c) generating said quantifiable signal; and
(d) detecting said quantifiable signal thereby detecting said polynucleotide sequence.

The method of the present invention employs, a plan or plastic substrate for fixing thereto and forming a double-stranded polynucleotide, one of the strands of said double-stranded polynucleotide being a non-radioactive chemically labeled polynucleotide or comprises a non-radioactive chemically-labeled nucleotide as a nucleotide component of said one strand. The substrate may be a transparent substrate or a translucent substrate.

The chemically-labeled nucleotide mentioned above may be the compound, 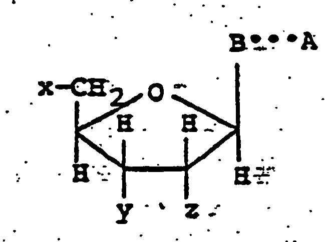 wherein B represents a purine, 7-deazapurine or pyrimidine moiety covalently bonded to the C^{1'}-position of the sugar moiety, provided that when B is purine or 7-deazapurine, it is attached at the N⁹-position of the purine or deazapurine, and when B is pyrimidine, it is attached at the N¹-position;
wherein A represents a moiety consisting of at least
three carbon atoms which is capable of forming a detectable complex with a polypeptide when the compound is incorporated into a double-stranded ribonucleotide acid, deoxyribonucleic acid duplex, or DNA-RNA hybrid;
wherein the dotted line represents a linkage or group joining B and A, provided that if B is purine, the linkage is attached to the 8-position of the purine, if B is 7-deazapurine, the linkage is attached to the 7-position of the deazapurine, and if B is pyrimidine, the linkage is attached to the 5-position of the pyrimidine; and
wherein each of x, y and z represents

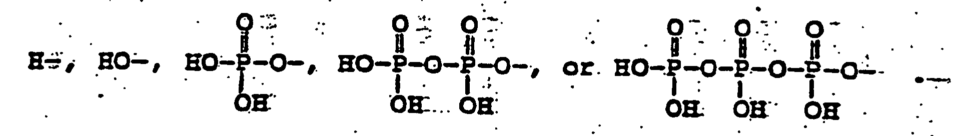

The chemically-labeled polynucleotide mentioned above may have the formula, 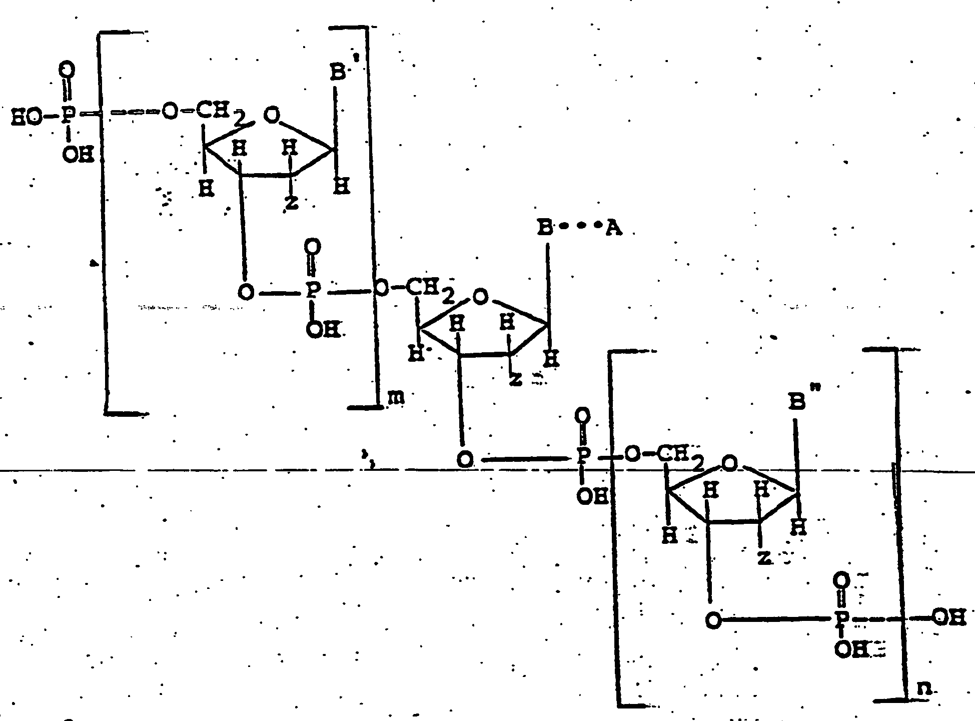 wherein each of B, B' and B" represents a purine, deazapurine, or pyrimidine moiety covalently bonded to the C^{1'}-position of the sugar moiety, provided, that whenever B, B' or B" is purine or deazapurihe, it is attached at the N⁹-position of the purine or deazapurine, and whenever B, B' or B" is pyrimidine; it is attached at the N¹⁻position;
wherein A represents a moiety consisting of at least three carbon atoms which is capable of forming a detectable complex with a polypeptide when the compound is incorporated into a double-stranded duplex formed with a complementary ribonucleic or deoxyribonucleic acid molecule;
wherein the dotted line represents a chemical linkage or group joining B and A, provided that if B is purine, the linkage is attached to the 8-position of the purine, if B is 7-deazapurine, the linkage is attached to the 7-position of the deazapurine, and if B is pyrimidine, the linkage is attached to the 5-position of the pyrimidine;
wherein Z represents H- or HO-; and
wherein m and n represent integers from 0 up to about 100,000.

chemically-labeled nucleotide may have the general formula,

P-S-B-Sig

wherein P is the phosphoric acid moiety, S the sugar or monosaccharide moiety, B being the base moiety, the phosphoric acid moiety being attached at the 3' and/or the 5' position of the sugar moiety when said nucleotide is a deoxyribonucleotide and at the 2', 3' and/or 5' position when said nucleotide is a ribonucleotide, said base being a purine or a pyrimidine, said base being attached fromt he N1 position or the N9 position to the 1' position of the sugar moiety when said base is a pyrimidine or a purine, respectively, and wherein said Sig is a chemical moiety covalently attached to the base B of said nucleotide, said Sig when attached to said base B being capable of signalling itself or making itself self-detecting or its presence known.

The chemically-labeled nucleotide may also have the general formula, 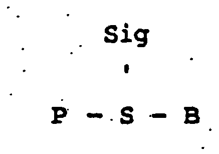 wherein.P is the phosphoric acid moiety, S the sugar moiety and B the base moiety, the phosphoric acid moiety being attached at the 2', 3' and/or 5' position of the sugar moiety, said base B being attached fromt he N1 position or the N9 position to the 1' position of the sugar moiety when said base is a pyrimidine or a purine, respectively, and wherein said Sig is a chemical moiety covalently attached to the sugar S, said Sig, when attached to said Sugar S, being capable of signalling itself or making itself self-detecting or its presence known.

The chemically-labeled nucleotide may also have the general formula, 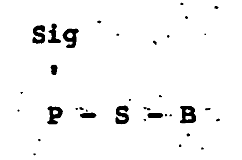 wherein P is the phosphoric acid moiety, S the sugar moiety and B the base moiety, the phosphoric acid moiety being attached to the 3' and/or the 5' position of the sugar moiety when said nucleotide is a deoxyribonucleotide and at the 2', 3' and/or 5' position when said nucleotide is a ribonucleotide, said base B being a purine or pyrimidine, said base B being attached from the N1 position or the N9 position to the 1' position of the sugar moiety when said base B is a pyrimidine or a purine, respectively, and wherein Sig is a chemical moiety covalently attached to the phosphoric acid moiety via the chemical linkage, 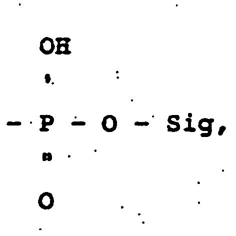 said Sig, when attached to said phosphoric acid moiety P being capable of signalling itself or making itself self-detecting or its presence known.

The chemically-labeled nucleotide may also have the general formula,

P - S - B - Sig

wherein P is the phosphoric acid moiety, S the sugar and monosaccharide moiety, B being the base moiety, the phosphoric acid moiety being attached to the 3' and/or the 5' position of the sugar moiety when said nucleotide is deoxyribonucleotide and at the 2', 3' and/or 5' position when said nucleotide is a ribonucleotide, said base being a purine or a pyrimidine, said base being attached from the N1 position or the N9 position to the Cl' position of the sugar moiety when said base is a pyrimidine or a purine, respectively, and wherein said Sig is a chemical moiety covalently attached to the base B of said nucleotide, said Sig being attached to the N⁶ or 6-amino group when said base B is adenine or the N² or 2-amino group when said base B is guanine or the N⁴ or 4-amino group when said base B is cytosine, said Sig when attached to said base B being capable of signalling itself or making itself self-detecting or its presence known. The chemically-labeled nucleotide may also have the general formula,

P-S-B,

wherein P is the phosphoric acid moiety, S the sugar or monosaccharide moiety and B the base moiety, said nucleotide having covalently attached to the P or S or B moiety a chemical moiety Sig, said Sig chemical moiety when attached to the P moiety, is attached thereto via the chemical linkage, 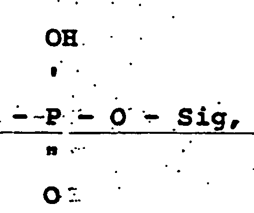 and when Sig is attached to the S moiety, the S moiety is a ribose group, said chemical moiety Sig when attached to said P, S or B being capable of signalling itself or making itself self detecting or its presence known.

The present invention provides in an embodiment a method for detecting the presence of a pathogen in a clinical sample suspected of containing said pathogen, said method comprising depositing said sample on an inert transparent or translucent glass or plastic support, treating said sample to affix genetic material of any of said pathogens present in said sample to said support in substantially single-stranded form, contacting said fixed single-stranded genetic material with a non-radioactive chemically-labeled probe having a nucleotide sequence of at least about 25 bases at least substantially complementary to a nucleotide sequence of a structural gene characteristic of said pathogen, said chemically labeled probe therein a chemically labeled nucleotide or polynucleotide, said contacting being under hybridizing conditions at a predetermined stringency and detecting duplex formation on said support by means of said chemically labeled probe. In said method for detecting the presence of a pathogen the chemically labeled nucleotide :
or polynucleotide has the above mentioned characteristics. The pathogen to be detected may be a streptococcus, a staphylococcus, a pneumococcus, a meninpococcus, a salmonella typhimurium or a clostridium botulinum.

In this method, a material or device for detecting or measuring a color change or chemical reaction colorimetrically or photometrically which is packaged in association with instructions for use in this method may be employed.

This method may also employ a transparent glass substrate prepared by treating the glass surface with aqueous nitric acid solution at a temperature of about the boiling point of the nitric acid solution, washing the resulting nitric acid-treated glass surface and, after drying the thus-treated glass surface, contacting the thus-treated glass surface with gamma-aminopropyltriethoxysilane, washing and drying the resulting treated glass surface and fixing DNA material thereto and being useful for the detection or measurement or a color change or a chemical reaction colorimetrically or photometrically whereby the substrate is packaged in association with instructions for use in this method. Also a glass or plastic substrate for fixing thereto and forming a double-stranded polynucleotide, one of the strands of said double-stranded polynucleotide being a non-radioactive chemically labeled polynucleotide or comprises a non-radioactive chemically-labeled nucleotide as a nucleotide component, of said one strand which is packaged in association with instructions for use in this method may be employed.

In a further embodiment of the method for detecting a polynucleotide sequence the substrate contains said double-stranded polynucleotide attached thereto, said double-stranded polynucleotide comprising said single-stranded non-radioactive chemically labeled polynucleotide or nucleotide, the other strand being an unlabeled single-stranded polynucleotide, said substrate being prepared by fixing said other single-stranded unlabeled polynucleotide to said substrate and then hybridizing to said unlabeled polynucleotide said one strand containing said chemically labeled polynucleotide or nucleotide. The single-stranded non-radioactive chemically labeled polynucleotide may contain at least 25 bases which are substantially complementary to the bases making up the other unlabeled single-stranded polynucleotide of said doublestranded polynucleotide,

In a further embodiment, the chemically labeled polynucleotide may be a polynucleotide coupled to or attached to a polypeptide. The polypeptide may be terminally attached to or ligated to said polynucleotide.

Moreover, the chemically labeled polynucleotide may comprise or have attached thereto an amino acid or polypeptide comprising a Sig chemical moiety covalently attached thereto, said Sig chemical moiety being capable of signalling itself or making itself self-detecting or its presence known whereby said Sig chemical moiety may comprise a saccharide component or a co-enzyme. The co-enzyme may be selected from the group consisting of thiamine pyrophosphate, flavine mononucleotide, flavine adenine dinucleotide, nicotinamide adenine dinucleotide, nicotinamide adenine dinucleotide phosphate, co-enzyme A, pyridoxyl phosphate, biotin, tetrahydrofolic acid, coenzyme B₁₂, lipoic acid and ascorbic acid. In a further embodiment, the chemically labeled polynucleotide may comprise or have attached thereto a monosaccharide or a polysaccharide comprising a Sig chemical moiety attached thereto, said Sig chemical moiety being capable of signalling itself or making itself self detecting or its presence known,
whereby said Sig chemical moirety may comprise a chelating agent or a co-enzyme.

The co-enzyme may be selected from the group consisting of thiamine pyrophosphate, flavine mononucleotide, flavine adenine dinucleotide, nicotinamide adenine dinucleotide, nicotinimide adenine dinucleotide phosphate, co-enzyme. A, pyridoxyl phosphate, biotin, tetrahydrofolic acid, co-enzyme B₁₂, lipoic acid and ascorbic acid.

In the method of the present invention, the double-stranded polynucleotide may be a double-stranded polyribonucleotide or a double-stranded polydeoxyribonucleotide or may comprise as one of the strands polydeoxyribonucleotide and the other strand a polyribonucleotide.

The glass or plastic substrate used in the method of the present invention may be transparent and adapted for the transmission of light therethrough for color observation or colorimetric determination of the duplex on said transparent substrate. Said transparent substrate may be glass. Or the substrate may be translucent and

adapted for the transmission of light therethrough for color observation or colorimetric determination of said duplex on said translucent substrate.

The substrate may be a planar substrate provided with a well therein wherein said duplex is fixed whereby said substrate carrying said duplex fixed to a well therein may be adapted for the transmission of light therethrough for the photometric or colorimetric determination of the duplex fixed within said well. The substrate may be a polystyrene, a polyethylene substrate or a polypropylene substrate.

The substrate may be an epoxy-coated subtrate or may have amino groups attached or fixed to the surface thereof. It may also provide a surface capable of fixing or immobilizing negatively charged polyelectrolytes thereon or provide a surface capable per se or untreated of fixing or immobilizing single-stranded or double-stranded polynucleotides thereto or permits the hybridization of a non-radioactive chemically-labeled polynucleotide to an unlabeled single-stranded polynucleotide or DNA fixed to said surface.

In a further embodiment, the present invention provides a method for determining the quantifiables presence of a selected genetic material which comprises treating a sample containing said selected genetic material so as to de-nature the same to provide a single strand thereof derived from said genetic material, fïxing or immobilizing the resulting single strand of genetic material to a glass or plastic surface, contacting under hybridizing conditions the thus-fixed single strand of genetic material with a non-radioactively chemically-labeled polynucleotide probe substantially complementary to the nucleotide components characterizing said genetic material to be determined and detecting duplex formation between said single strand of genetic material and said non-radioactive chemically-labeled polynucleotide probed by photometric techniques.

The surface used therein may be a transparent surface or a translucent surface and is preferably a glass surface. Preferably, the surface is a glass surface and said single-stranded genetic material is fixed to a well provided on said surface, said surface being adapted for the transmission of light therethrough for the photometric or colorimetric determination of duplex formation between said single-stranded genetic material and said polynucleotide probe. The duplex formation between said single strand of genetic material and said non-radioactive, chemically-labeled polynucleotide probe may be detected or determined by enzyme linked immunosorbent assay (ELISA). The detection of duplex formation between said single strand of genetic material and said non-radioactive, chemically-labeled polynucleotide probe may furthermore be determined by forming a complex with a chemical label provided on said polynucleotide probe, said complex comprising an enzyme, and eliciting the presence of said enzyme-containing complex attached to said chemical label by bringing said enzyme-containing complex attached to said polynucleotide probe into contact with a substrate which undergoes a chemical or color change when in contact with said enzyme. The duplex or double strand formation or hybridization between said single strand of material and said non-radioactive chemically-labeled polynucleotide probe may also be determined by providing said chemical label attached to said polynucleotiode probe, before or after duplex formation, with a chelating agent and eliciting the presence of said chelating agent by contacting said chelating agent with a substrate which undergoes a chemical reaction or color change when in contact with said chelating agent. The presence of said chelating agent may be elicited by contacting said chelating agent with a substrate. which undergoes a chemical raction or a color change when in contact with said chelating agent. The color change is photometrically determined and is indicative of the amount of chelating agent fixed to the resulting formed duplex.

The glass surface used in the present invention for fixing genetic DNA material thereto is prepared by treating the glass surface with aqueous nitric acid solution at a temperature of about the boiling point of the nitric acid solution, washing the resulting nitric acid-treated glass surface and, after drying the thus-treated glass surface, contacting the thus-treated glass surface with gamma-aminopropyl triethoxysilane, washing and drying the resulting treated glass surface and fixing DNA material thereto. In another embodiment, the glass surface may be a transparent glass surface suitable for fixing genetic DNA material thereto, said having attached or fixed thereto amino groups. A surface adapted for the fixing of genetic material thereto may be prepared by contacting said surface with the amine and the hardener components of an epoxy glue to form a coating of epoxy glue thereon. A further method of treating a glass surface for the fixing genetic DNA material thereto comprises contacting said glass surface with an organosilane, said organosilane providing an organic functional group at one end and a silyl alkoxy group at the other end under conditions so as to fix the silyl alkoxy group portion of the organosilane to said glass surface.

In the method of the present invention, a planar form transparent glass plate provided with rows of wells or depressions formed on the surface thereof may be used for fixing thereto and forming double-stranded polynucleotide, one of the strands of said double-stranded nucleotide being a non-radioactively chemically-labeled polynucleotide or comprises a non-radioactively chemically-labeled nucleotide as a nucleotide component of said one strand The substrate may also be a cuvette having a planar surface adapted for the transmission of light therethrough or the substrate is adapted for the transmission of light perpendicularly through the substrate or transversely through the system.

In the method for determining the quantifiable presence of a selected genetic material, the detection of duplex formation by the said single-strand of genetic material and said non-radioactive chemically-labeled polynucleotide probe is determined by forming a complex with a chemical label providing on said poly-nucleotide probe, said complex comprising a component capable of signalling or eliciting its presence by spectrophotometric or colorimetric means.

In this method of the present invention, a transparent glass container or cuvette provided with planar walls may be used for fixing to and forming a double-stranded polynucleotide on the interior surface or the container, one of the strands of said double-stranded polynucleotide being a non-radioactively chemically-labeled polynucleotide or comprises a non-radioactively chemically-labeled nucleotide as a nucleotide component of said one-strand.

The present invention also provides a method for detecting the presence or identity of genetic DNA material which comprises depositing a sample of genetic material on an inert transparent or translucent glass or plastic support surface, treating said sample to affïx said genetic DNA material to said support in substantially single-stranded form, contacting said fixed single-stranded genetic DNA material with a non-radioactive chemically-labeled probe having a nucleotide sequence of at least about 25 bases, at least substantially complementary to the nucleotide sequence contained in said genetic DNA material, said chemically-labeled probe having therein a chemically-labelled nucleotide as defined above said contacting being under hybridizing conditions at a pre-determined stringency and detecting duplex formation of said genetic DNA material fixed to said support surface: by means of said chemically-labeled probe.

The chemical label moiety of the non-radioactive chemically labeled nucleotide used in the method of the present invention comprises an enzyme component effective upon contact with a chromogen to produce an insoluble colored precipitate or product so as to indicate, define or elicit the presence and/or location of said double-stranded polynucleotide on said substrate.

In a further embodiment, the present invention provides a method for detecting or eliciting the presence of a pathogen in a clinical, sample suspected of containing said pathogen, said method comprising depositing said sample on a glass or plastic substrate, treating said sample to affix genetic material of any of said pathogens present in said sample to said substrate in substantially single-stranded form, contacting said fixed single-stranded genetic material with a non-radioactive chemically labeled polynucleotide probe having a nucleotide sequence substantially complementary to the nucleotide sequence of said single-stranded genetic material affixed to said substrate, said contacting being carried out under hybridizing conditions at a predetermined stringency - to effect duplex formation between said single-stranded genetic material and said non-radioactive chemically labeled polynucleotide probe, adding or attaching to the chemical label moiety of said non-radioactive chemically labeled polynucleotide probe a complex comprising an enzyme component effective upon contact with a chromogen to produce an insoluble colored precipitate or product and bringing the resulting formed duplex comprising said chemically labeled polynucleotide probe and said single stranded genetic material into contact with said chromogen to produce said insoluble colored precipitate or product, thereby detecting or eliciting or defining on said substrate the presence of said single-stranded genetic material derived from said pathogen.

In the method of the present invention the substrate contains said double-stranded polynucleotide fixed thereto, said double-stranded polynucleotide comprising said single-stranded non-radioactive chemically labeled polynucleotide or nucleotide, the other strand being an unlabeled single-stranded polynucleotide, said substrate being prepared by fixing said other single-stranded unlabeled polynucleotide to said substrate, contacting said fixed unlabeled single-stranded polynucleotide with said single-stranded non-radioactive chemically labeled polynucleotide under hybridizing conditions to effect formation therefrom of said double-stranded polynucleotide, the chemical label moiety of said non-radioactive chemically labeled nucleotide comprising an enzyme component effective upon contact with a chromogen to produce an insoluble colored precipitate or product so as to indicate, define or elicit the presence and/or location of said double-stranded polynucleotide of said substrate.

Furthermore, a method for determining the presence of a selected genetic material is provided which comprises treating a sample containing said selected genetic material so as to denature the same to provide a single strand thereof derived from said genetic material and characterizing said selected genetic material, fixing the resulting single strand of genetic material to a glass or plastic substrate, contacting under hybridizing conditions the thus-fixed single strand of genetic material with a non-radioactive chemically labeled single-stranded polynucleotide probe having a nucleotide sequence substantially complementary to the nucleotide components or sequence characterizing said selected genetic material to be determined, adding to the chemical label moiety of said non-radioactive chemically labeled polynucleotide probe an enzyme component effective upon contact with a chromogen to produce an insoluble colored precipitate or product and bringing the resulting formed duplex comprising said chemically labeled polynucleotide probe and said single-stranded genetic material into contact with said chromogen to produce said insoluble colored precipitate or product, thereby determining or eliciting or defining on said substrate the presence of said single stranded genetic material.

Furthermore a, method for determining the presence of a selected single-stranded genetic material which has been fixed to a glass or plastic substrate, is provided which comprises bringing said fixed single-stranded genetic material into contact under hybridizing conditions with a chemically labeled single-stranded polynucleotide probe having a nucleotide sequence sufficiently complementary with the nueleotide sequence characterizing said selected single-stranded genetic material to effect hybridization of said single-stranded polynucleotide probe with said single-stranded genetic material adding to the chemical label moiety of said chemically-labeled polynucleotide probe for attachment thereto a chemical component effective on contact with a chromogen to produce an insoluble colored precipitate or product and bringing the resulting formed hybrid comprising said chemically-labeled polynucleotide probe and said single-stranded genetic material into contact with said chromogen to produce said insoluble colored precipitate or product, thereby determining or eliciting or defining on said substrate the presence of said selected single-stranded genetic materials. The chemical component may comprise an enzyme complex which attaches to or fixes to said chemical moiety of said chemically-labeled polynucleotide probe. The enzyme may be alkaline phosphatase, horseradish peroxidase, glucose oxidase and β-galactosidase.

The single-stranded genetic material may be derived from a clinical sample suspected of containing a pathogen or from a gene or fragment thereof.

In the method of the present invention, the chemical label, moiety of said non-radioactive chemically-labeled polynucleotide or nucleotide comprises attached thereto a chemical component effective upon contact with a chromogen to produce an insoluble colored precipitate or product so as to indicate, define or elicit the presence and/or location of the duplex on the substrate. Thereby, the chemical component may comprise an enzyme selected from the group consisting of alkaline phosphatase, horseradish peroxidase glucose oxidase, and β-galactosidase, and wherein said substrate comprises nitrocellulose.

The method in accordance with the present invention may employ a device for detecting or measuring a color change or chemical reaction colorimetrically or photometrically which comprises a transparent glass or plastic substrate which has associated with or fixed thereto a single stranded chemically labeled DNA probe hybridized to a complementary single stranded DNA material, said chemical label having an enzyme complex attached thereto, means for adding a chemical substrate to contact said enzyme complex attached to said chemical label of said hybridized single stranded DNA probe associated with or fixed to said transparent substrate so.as to generate by reaction between said enzyme complex and said chemical substrate a color change or a photometrically detectable chemical reaction, means for illuminating or passing light onto or through said transparent substrate containing said enzyme complex involved in said color change or chemical reaction comprising means for colorimetrically or photometrically detecting said color change or photometrically detectable chemical reaction brought about by contact between said enzyme complex and said chemical substrate. The means for illuminating or passing light onto or through said transparent substrate containing said enzyme complex involved in said color change or said chemical reaction may include means for quantitatively measuring said color change or said photometrically detectable chemical reaction.

The device may be packaged in association with instructions for use in the method for detecting the presence of a pathogen in a clinical sample suspected of containing said pathogen, said method comprising depositing said sample on an inert transparent or translucent support, treating said sample to affix genetic material of any of said pathogens present in said sample to said support in substantially single-stranded form, contacting said fixed single-stranded genetic material with a non-radioactive chemically-labeled probe having a nucleotide sequence of at least about 25 bases at least substantially complementary to a nucleotide sequence of a structural gene characteristic of said pathogen, said chemically labeled probe having therein a chemically labeled nucleotide 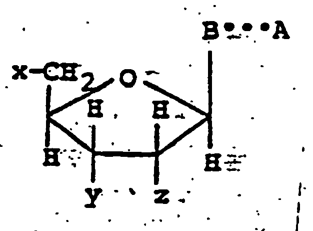 as defined above said contacting being under hybridizing conditions at a Predetermined stringency and detecting duplex formation on said support by means of said chemically labeled probe.

The device may also employed a transparent substrate prepared by contacting the surface of the substrate with the amine and the hardener components of an epoxy glue to form a coatings of epoxy glue thereon, the substrate being useful for detecting or measuring a color change or chemical reaction colorimetrically or photometrically. The substrate is packaged in association with instructions and may be in accordance with the method for detecting the presence or identity of genetic DNA material which comprises depositing a sample of genetic material on an inert transparent or translucent glass or plastic support surface, treating said sample to affix said genetic DNA material to said support in substantially single-stranded form, contacting said fixed single-stranded genetic DNA material with a non-radioactive chemically-labeled probe having a nucleotide sequence of at least about 25 bases, at least substantially complementary to the nucleotide sequence contained in said genetic DNA material, said chemically-labeled probe, having therein a chemically-labeled nucleotide as defined directly above, said contacting being under hybridizing conditions at a pre-determined stringency and detecting duplex formation of said genetic DNA material fixed to said support surface by means of said chemically-labeled probe.

The above substrate is also in general useful in the above method.

It is known to fix enzymes and the like to siliceous materials and to detect antibodies and the like involving attachments of such materials as enzymes, antibodies and antigens to various substrates, such as siliceous substrates. See for example U.S. patents 3,669,841, 3,715,278, 3,949,064, 4,001,583, 4,059,685, 4,120,945 and 4,280,992. .In the practices of this invention, it is highly desirable that the fixing of the denatured single-stranded genetic DNA material derived from the DNA material to be identified be rapidly fixed to the substrate, such as a transparent substrate, e.g. a glass surface. Rapid fixing of single-stranded DNA material to.a transparent glass substrate would permit rapid testing of numerous samples involving ELISA techniques. For example, glass plates provided with an array of depressions or wells therein would have samples of the various denatured genetic materials to be identified deposited therein and the single-stranded DNA material therein fixed to the surfaces of the wells. Thereupon, DNA probes provided with a non-radioactive chemical label are deposited in each of the wells for hybridization to any complementary single-stranded DNA material therein. After washing to removes any non-hybridized probe, the presence of any hybrid DNA material containing the single-stranded DNA material to be identified and the non-radioactive chemically-labeled probe is detected, as described herein, involving the addition of an enzyme linked antibody or other suitable entity for attachment to the chemical label of the probe. Subsequently a suitable substrate is added to elicit a color change or chemical reaction which could then be measured colorimetrically or photometrically.

To effect rapid fixing of DNA material, such as denatured single-stranded DNA, to glass, the glass is prepared or pretreated by heating or boiling the glass, such as a borosilicate glass, for a sufficient period of time, e.g. about 45 minutes, in the presence of dilute, e.g. 5 percent, aqueous nitric acid. This pretreatment with nitric acid serves to leach out boron residues from the glass surface. The treated glass is then washed or rinsed with water, preferably with distilled water, and dried, such as at a temperature of about 115°C, for about 24 hours. A 10 percent solution of gamma-aminopropyltriethoxysilane prepared by dissolving the above-identified silane in distilled water followed by addition of 6N hydrochloric acid to a pH of about 3.45, is then applied to the glass surface, and the glass surface is then incubated in contact with the above-identified silane solution for about 2-3 hours at a temperature of about 45°C. The glass surface is then washed with an equal volume of water and dried overnight at a temperature of about 100°C. The resulting treated glass surface has available alkylamine provided on the surface thereof suitable for immobilizing or fixing any negatively charged polyelectrolytes applied thereto. In connection with the above, see Weetal, H.H. and Filbert, A.M., "Porous Glass for Affinity Chromatography Applitations", Methods in Enzymology, Vol. XXXIV, Affinity Techniques Enzyme Purification: part B, pp. 59-72, W.B. Jakoby and M. Wilchek, eds.

To illustrate the practices of this invention, various DNA materials were prepared. For example, bacteriophage T₄ DNA was isolated from E. coli CR63 cultures infected with phage T₄ AM82 [44⁻62⁻] and purified to be free of chromosomal DNA. Highly purified calf thymus DNA was obtained from a biological supply service. Bacteriophage lambda was obtained by heat induction of E. coli strain W3350 lysogenic for λC₁ 857 phage and was employed for the preparation of phage lambda DNA. Also, radioactive. DNA was prepared by nick translation with [³H] dATP and phage lambda DNA was also nick translated with maltose-triose dUTP to introduce glucosyl or saccharide residues into the DNA. Other reagents were obtained or prepared, for example, lectins which readily and eagerly form a complex with glucosyl groups. Specifically Concanavalin A [ConA] was obtained and solubilized in 2.0 M NaCl at a concentration of 50 mg/ml. Also, fluorescein-labeled ConA was prepared by reacting ConA with fluorescein isothiocyanate at a FITC to protein molar ratio of 3 in 0.1 M sodium borate solution at a pH of 9.2 and at a temperature of 37°C for 60 minutes. Any unreacted FITC was removed by gel filtration on Sephadex G-50.

A glass surface treated as described hereinabove was employed in the detection of lectin-binding to glucosylated DNA. In this procedure, glucosylated DNA [T₄ DNA] or non-glucosylated DNA [calf thymus DNA] was delivered in 100 µl portions to treated glass tubes in triplicate sets. After 15-30 minutes at room temperature, the solution was removed and the tubes rinsed generously with PBS·Mg⁺⁺. buffer [100 mM. Na-K-Po₄, pH 6. 5, 150 mM NaCl and 10 mM MgCl₂]. One set of tubes was checked for the presence of DNA by staining with ethidium bromide [100 µl of 1 mg/ml solution, 30 minutes in the dark, at room temperature]. The staining solution was removed and the tubes rinsed and checked under UV light. To another set of tubes there was delivered fluorescent-labeled ConA. [100 µl of 0.1 mg/ml in PBS•Mg⁺⁺ buffer]. After 60 minutes at room temperature, the solution was removed and the tubes were rinsed and checked under UV light.

To the third set of tubes was delivered 100 µl of un-labeled ConA in PBS•Mg⁺⁺ buffer. After 60 minutes at room temperature, the tubes were rinsed free of ConA with 0.2 M Imidazole buffer pH 6.5.

Acid phosphatase was then added [0.005 units in 100 µl at 0.2 percent phosphatase-free BSA] and the tubes were incubated at room temperature for 30 minutes. After rinsing with 0.15 M NaCl (to remove any unbound enzyme) substrate [para-nitrophenylphosphate 0.1mM in 0.2M Imidazole pH 6.5] was added and incubation continued: for 60 minu at 37^{o}C. The enzyme reaction was terminated by adding 1.0 ml of 0.5 percent sodium bicarbonate and absorbance was determined at A₃₀₀.

The resulting observed test results indicated that both glucosylated and non-glucosylated DNA bound to the activated glass surface by the observed red fluorescence characteristic of ethidium bromide. Also, ConA bound only to glucosylated DNA as noted by the green fluorescence in tubes containing T₄ DNA, but not in tubes which had calf thymus DNA. Further, acid phosphatase, which is a glyco-protein, gave positive reaction only in tubes containing T₄ DNA and ConA, but was washed off from the tubes which contained only ConA or ConA and calf thymus DNA.

Activated glass tubes were also employed in the detection of lectin-binding to glucosylated DNA probes hybridized to DNA already immobilized on glass surface. In these test, phage lambda DNA was immobilized on glass surface. After rinsing with buffer, the tubes were coated with 100 µl of coating, solution [50 percent formamide, 5X SSC, 100 µg salmon sperm DNA, 0.2 percent polyvinyl pyrrolidone, 0.1 percent Triton X-100, 0.2 percent BSA and 0.05 percent SDS] at 42°C for 90-120 minutes. The coating solution was removed and the surface was covered with 100 µl of costing solution containing glucosylated nick-translated DNA probe. The probe had been denatured at 80°C for 3 minutes and rapidly cooled in ice bath immediately before use. The tubes were then incubated at 42°C for 24 hours. The solution was removed and tubes were rinsed with PBS**·**Mg⁺⁺ buffer. The lectin-enzyme detection system was delivered as described before. The results indicated that acid phosphatase was hot washed off from the tubes which contained glucosylated DNA probe, whereas tubes containing non-glucosylated probe did not show any enzyme activity.

In these tests, the glucosyl (monosaccharide) moiety of the glucosylated DNA probe serves the non-radioactive chemical label and reacts with or is strongly attracted to the lectin; ConA, making up the combination lectin-enzyme (acid phosphatase) probe detection system.

In the above test employing glucosylated DNA as a probe, wherein the glucosyl- or monosaccharide moiety of the glucosylated DNA probe serves as the non-radioactive chemical label, comparable results are also achievable in the practice of this invention employing a biotin-labeled DNA probe. When biotin is employed as the non-radioactive chemical label of the DNA probe, and since avidin is strongly reactive with or strongly bonds to biotin, the presence of the biotin-labeled DNA probe would be elicited or detected by means of avidin or streptavidin-labeled enzyme. For example, a biotin-labeled DNA probe would readily be detected by an enzyme complex of the character avidin-biotin-alkaline phosphatase. More specifically, the presence of the biotin-labeled DNA probe would readily be elicited or detected by contacting the hybrid containing the biotin-labeled probe with the enzyme complex avidin-biotin-alkaline phosphatase, followed by bringing the resulting biotin-labeled DNA probe having attached thereto the avidin-biotin-alkaline phosphatase complex into contact with a suitable substrate which by color reaction or precipitate formation brought about by the alkaline phosphatase would be readily noticed or capable of being determined, both qualitatively and quantitatively, by photometric and/or colorimetric means in accordance with ELISA techniques. If desired, instead of an avidin-biotin-enzyme complex, there could be used an antibody to biotin for attachment to the biotin moiety of the biotin-labeled DNA probe, followed by a complex comprising anti-antibody-enzyme in the manner described hereinabove.

The advantages of the practices of this invention are also obtainable when the DNA probe is fixed to or hybridized to a plastic surface, such as a polystyrene surface. When a plastic surface is employed, it is sometimes desirable, in order to increase the effectiveness or uniformity of the fixing of DNA applied thereto, including the non-radioactive chemically-labeled DNA probe, to treat the plastic surface, such as a polystyrene surface, to enhance and otherwise improve the fixing or immobilization of genetic material thereon, such as single-stranded denatured DNA or a non-radioactive chemically-labeled probe or hybrid DNA containing the DNA probe. It has been found, for example, that the adherence or fixing of DNA to a polystyrene surface is improved by treating the surface, as indicated hereinabove, with an amino-substituted hydrophobic polymer or material, such as duodecadiamine. Another technique for improving the fixing or uniformity of the plastic surface for fixing DNA involves treatment of the surface with polylysine (PPL).

In tests involving the fixing of DNA to a plastic surface, biotinylated DNA was denatured and aliquoted into Dynatech, Immulon II™ removeable wells. Samples were allowed to dry onto the plastic surface at 37°C. The amount of bound b-DNA was determined by sequential addition of goat anti-biotin antibody and rabbit anti-goat antibody complexed to alkaline phosphatase, followed by development with p-nitrophenyl phosphate in diethanolamine buffer, pH 9.6. Enzymatic activity was monitored at 405 nm utilizing the automatic Dynatech Micro ELISA Scanner. This procedure enables the investigator to quantitate the amount of bound DNA and circumspectively the degree of biotinylation.

To increase the sensitivity of detection, a fluorogenic substrate, such as 4-methylumbelliferyl-phosphate, or its analogues, with companion enzymes, may be used.

Additional tests were carried out wherein several aliquots of denatured adenovirus 2 DNA were bound to polystyrene plates as described above. After blocking with Denhardt's formamide blocking buffer, several biotinylated probes were hybridized to the immobilized DNA; these were B-adeno-2-DNA and lambda DNA. To one set of immobilized DNA, no probe was added. The extent of hybridization was determined by means of the antibody-enzyme reaction as described above. It was observed that only the homologous adeno-2 probe hybridized. This technique demonstrated that in vitro hybridization under these conditions is specific and can be monitored quantitatively.

Polystyrene from various batches or sources exhibits different binding capacities. Previous experiments had demonstrated that addition of duodecadiamine (DDA) to polystyrene resulted in a uniform binding coefficient of polystyrene plates of different batches.

In further tests, radioactively-labeled, non-biotinylated denatured DNA [2000 ng to 5 ng] was applied to DDA-coated polystyrene plates. The test samples or plates were not allowed to dry. After incubation at 37°C for 30 minutes, 1 hour, 2 hours, 3 hours, 4 hours, and 18 hours, samples were counted. Binding was maximal after 2 hours of incubation, however, 50 percent of the originally applied DNA bound regardless of the concentration, thereby indicating that there is an equilibrium between, bound and unbound DNA.

In other tests, polystyrene microfilter wells were nitrated using a procedure of Filipsson and Hornby [Biochem J. 120 215 (1970)]. The polystyrene wells were immersed for 20 minutes in a mixture of concentrated nitric and sulfuric acid [41 percent, v/v] cooled to 0°C. The wells were then washed thoroughly with water and subsequently heated to 70°C in a 6 percent solution of sodium dithionate in 2M potassium hydroxide. After 4 hours, the wells were washed thoroughly with 0.5M hydrochloric acid and distilled water.

To produce 6-aminohexane linked polystyrene, 6-aminocaproic acid-N-hydroxysuccinimide ester•hydrobromide [5 mg thereof dissolved in 0.2M dimethylformamide prepared by reacting 6-aminocaproic acid•hydrobromide with N-hydroxysuccinimide and dicyclohexyl carbodiimide in dimethylformamide and recrystallized from isopropylalcohol] was added to 0.1M sodium borate [0.4 ml]. Amino-derivitized polystyrene microfilter wells filled with this solution were allowed to react at room temperature for 4 hours and then washed thoroughly with distilled water. The resulting treated wells absorbed ³H-labeled DNA from aqueous solution at pH less than 9.5.

As indicated hereinabove siliceous substrates, such as glass substrates, and plastic substrates, such as polystyrene substrates, are improved with respect to the capability of fixing or immobilization of DNA thereto by treatment with or by providing thereon a coating of an epoxy resin. For example, glass and polystyrene surfaces are improved with respect to the fixing or immobilization of DNA thereon by treatment of glass or polystyrene surfaces with commercially available epoxy glues, such as a solution of epoxy glue in ethanol [1 percent w/v]. These epoxy solutions are applied to the glass, e.g. Pyrex, glass surfaces or polystyrene surfaces or wells, and the solvent, ethanol, evaporated thereupon at a temperature of 37°C, thereby providing a polyamine polymeric coating on the thus-treated surface. These surfaces were found to absorb ³H-labeled DNA from aqueous solution at pH less than 9.5.

The methods described hereinabove with respect to ELISA-like techniques for the colorimetric or photometric determination of the hybridized probes involve enzyme-linked reagents which produce a color change in a substrate or a precipitate. Various combinations of enzymes and color-producing substrates may be employed. See for example accompanying tables. Table I and Table II, showing the combination of enzymes and chromogens. In Table I, the chromogen found in the substrate is reactive with the enzyme employed to elicit or determine the presence of the non-radioactive chemically-labeled probe. The superscript notation (F) indicates that the chromogen fluoresces. In accompanying Table II, there are set forth those chromogens which produce an insoluble product, such as when employed with the avidin/streptavidin system. These combinations of enzyme-chromogen are particularly useful in ELISA techniques for the determination, both qualitatively and quantitatively, of the hybrid DNA or other genetic material containing the special non-radioactive chemically-labeled probe in accordance with this invention.

**TABLE I CHROMOGEN**

| **ENZYME** | |
|---|---|
| 1a. alkaline phosphatase | 4-Methylumbelliferyl phosphate ^{(F)} |
| b. acid phosphatase | bis (4-Methylumbelliferyl phosphate.) ^{(F)} 3-0-methylfluorescein. 'Flavone-3-diphosphate triammorium^{(F)} salt p-nitrophenyl phosphate 2Na. |
| 2. peroxidase | Tyramine hydrochloride^{(F)} 3-(p-hydroxyphenyl) Propionic acid^{(F)} p-Hydroxyphenethyl alcohol ^{(F)} 2,2'-Azino-Di-3-Ethylbenztbiazoline sulfonic acid (ABTS) ortho-phenylenediamine 2HCl o-dianisidine 5-aminosalicylic acid p-cresol^{(F)} 3,3'-dimethyloxybenzidine 3-methyl-2-benzothiazoline hydrazone tetramethyl benzidine |
| 3. β-D-galactosidase | 0-nitrophenyl β-D-galactopyranoside 4-methylumbelliferyl-β-D-galactoside |
| 4. glucose-oxidase | ABTS |

**TABLE II CHROMOGEN (insoluble product)**

| **ENZYME** | |
|---|---|
| 1. alkaline phosphates | 6-bromo-3-hydroxy-2-haphthyl-0 anisidine+ Past Violet B salt. 2,2'-dimethyl-3-hydroxy-2-naphthalide + Fast Red salt. flavone-3-diphosphate ammonium salt + AlCl₃ ^{(F)} 5-bromo-4-chloro-3-indolylphosphate + tetrazolium, nitro BT. |
| 2. Horseradish peroxidase | H₂O₂+ diaminobenzidine H₂O₂ + tetramethylbenzidine |
| 3. Glucose oxidase | glucose + thiazolyl blue |
| 4. β-galactosidase | 2-(β-D-galactosidoxy) naphthol AS-LC+ Fast Violet B salt. Naphthol-AS-B1-B-0-galactopyranoside + Past Blue BBN salt. |

## Claims

1. A method for detecting a polynucleotide sequence which comprises:
(a) fixing or immobilizing said polynucleotide sequence to a glass or plastic substrate such that the polynucleotide sequence is in a single-stranded form and is capable of hybridizing to complementary nucleic acid sequences;
(b) forming a duplex comprising said single-stranded polynucleotide sequence hybridized to an oligo- or polynucleotide probe, said probe having attached thereto a non-radioactive chemical label comprising a signalling moiety capable of generating a quantifiable signal detectable by means selected from photometric techniques, spectrophotometric techniques, visually, colorimetric techniques, chemiluminescent techniques, fluorometric techniques and immunofluorometric techniques;
(c) generating said quantifiable signal, and
(d) detecting said quantifiable signal thereby detecting said polynucleotide sequence.

2. The method of claim 1, wherein said substrate comprises a surface that is capable per se, or with or without treatment, of fixing or immobilizing a single-stranded or double-stranded polynucleotide thereto and still permits hybridization of a non-radioactively chemically-labelled polynucleotide to the fixed or immobilized polynucleotide.

3. The method of claim 1 or 2, wherein the generation of said signal comprises the enzymatic generation of a product that can be measured visually, colorimetrically, chemiluminescently, fluorometrically, immunofluorometrically, spectrophotometrically, or photometrically.

4. The method of claim 1 or 3, wherein the substrate comprises a transparent or.translucent surface adapted to permit detection by means selected from photometric techniques, spectrophotometric techniques, visually, colorimetric techniques, chemiluminescent techniques, fluorometric techniques and immunofluorometric techniques.

5. The method of any one of claims 1 to 4, wherein light is transmitted through the substrate for colorimetric or photometric determination of said signal.

6. The method of any one of claims 1 to 5, wherein said substrate comprises an epoxy-coated substrate.

7. The method of any one of claims 1 to 5, wherein said substrate comprises polystyrene, polyethylene or polypropylene.

8. The method of any one of claims 1 to 7. wherein detection of said quantifiable signal provides qualitative determination of the golynucleotide sequence; and quantitative determination of the polynucleotide sequence.

9. The method of any one of claims 1 to 8, wherein said polynucleotide sequence is associated with said substrate and said substrate is present in a device.

10. The method of any one of claims 1 to 9, wherein said signalling moiety includes a co-enzyme.

11. The method of claim 10, wherein said co-enzyme is selected from the group consisting of thiamine pyrophosphate, flavine mononucleotide, flavine adenine dinucleotide, nicotinamide adenine dinucleotide, nicotinamide adenine dinucleotide phosphate, coenzyme A, pyridoxyl phosphate, biotin, tetrahydrofolic acid, coenzyme B₁₂, lipoic acid and ascorbic acid,

12. The method of any one of claims 1 to 9, wherein said signalling moiety comprises a chelating agent.

13. The method of any one of claims 1 to 12, wherein said polynucleotide. sequence is fixed or immobilized to well provided on the surface, said surface being adapted for the transmission of light therethrough for photometric or colorimetric determination of said signal.

14. The method of any one of claims 1 to 13, further comprising one or more washing steps.

15. A method for determining the quantifiable presence of a selected genetic material which comprises treating a sample containing said selected genetic material so as to denature the same to provide a single strand thereof derived, from said genetic material fixing or immabitizing the resulting single strand of genetic material to a glass or plastic surface, contacting under hybridizing conditions the thus-fixed or immobilized single strand of genetic material with a non-radioactively chemically-labelled polynucleotide probe substantially complementary to nucleotide components characterizing said genetic material to be determined, and detecting duplex formation between said single strand of genetic material and said non-radioactively chemically-labelled polynucleotide probe by photometric techniques.

16. A method for determining the quantifiable presence of a selected genetic material which comprises treating a sample containing said selected genetic material so as to denature the same to provide a single strand thereof derived from said genetic material fixing or immobilizing the resulting single strand of genetic material to a glass or plastic surface, contacting under hybridizing conditions the thus-fixed or immobilized single strand of genetic material with a non-radioactively chemically-labeled polynucleotide probe substantially complementary to nucleotide components characterizing said genetic material to be determined, generating a detectable quantifiable signal by enzyme linked immunosorbent assay technique, and detecting duplex formation between said single strand of genetic material and said non-radioactively chemically-labeled polynucleotide probe by detecting said quantifiable signal.

17. The method of claim 15 or 16, wherein light is transmitted through said surface for determination of said duplex formation.

18. The method of claim 17, wherein said single-stranded genetic material is fixed or immobilized to a well provided on said surface, said surface being adapted for the transmission of light therethrough for the photometric or colorimetric determination of duplex formation between said single-stranded genetic material and said polynucleotide probe.

19. The method of any one of claims 15 or 17 to 18, wherein said duplex formation between said single strand of genetic material and said non-radioactively chemically-labelled polynucleotide probe is detected or determined by enzyme linked immunosorbent assay techniques.

20. The method of any one of claims 15 to 19, wherein detection of duplex formation between said single strand of genetic material and said non-radioactively chemically-tabelled polynucleotide probe is determined by forming a complex with a chemical label provided on said polynucleotide probe, said complex comprising an enyzme, and eliciting the presence of said enzyme-containing complex attached to said chemical label by bringing said enzyme containing complex attached to said polynucleotide probe into contact with a substrate which undergoes a chemical reaction or color change when in contact with said enzyme.

21. The method of any one of claims 15 to 18, wherein duplex or double strand formation or hybridization between said single strand of material and said non-radioactively chemically-labeled polynucleotide probe is determined by providing before or after duplex formation, a chelating agent to said chemical label attached to said polynucleotide probe, and eliciting the presence of said chelating agent by contacting said chelating agent with a substance which undergoes a chemical reaction or color change when in contact with said chelating agent.

22. The method of claim 21, wherein said color change is photometrically or colorimetrically determined and is indicative of the amount of chelating agent bound or attached to the resulting formed duplex.

23. A method for detecting a polynucleotide sequence which comprises:
(a) fixing or immobilizing said polynucleotide sequence to a glass or plastic substrate such that the polynucleotide sequence is in a single-stranded form and is capable of hybridizing to complementary nucleic acid sequences;
(b) forming a duplex comprising said single-stranded polynucleotide sequence hybridized to an oligo- or polynucleotide probe, said probe having attached thereto a non-radioactive chemical label comprising a signaling moiety capable of generating a soluble signal;
(c) generating said soluble signal; and
(d) detecting the generated soluble signal, thereby detecting said polynucleotide sequence.

24. A method for detecting a polynucleotide sequence which comprises: -
(a) forming a duplex comprising the polynucleotide sequence in single-stranded form hybridized to an oligo- or polynucleotide probe, said probe having attached thereto a non-radioactive chemical label comprising a signalling moiety capable of generating a quantifiable signal:
(b) fixing or immobilizing said duplex to a glass or plastic substrate such that the duplex is capable of generating a quantifiable signal;
(c) generating a quantifiable signal detectable by means selected from photometric techniques, spectrophotometric techniques, visually, colorimetric techniques, chemiluminescent techniques, fluorometric techniques and immunofluorometric techniques; and
(d) detecting said quantifiable signal thereby detecting said polynucleotide sequence.

25. The method of claim 24, wherein said generating of said quantifiable signal is by enzyme linked immunosorbent assay techniques.

## Patentansprüche

1. Verfahren zum Nachweis einer Polynucleotidsequenz, umfassend:
(a) Fixieren oder Immobilisieren der Polynucleotidsequenz an ein Glas- oder Kunststoffsubstrat, so dass die Polynucleotidsequenz in Einzelstrangform vorliegt und an komplementäre Nucleinsäuresequenzen hybridisieren kann;
(b) Bilden einer Duplex, die die einzelsträngige Polynucleotidsequenz hybridisiert an eine Oligo- oder Polynucleotidsonde umfasst, wobei an die Sonde ein nicht-radioaktiver chemischer Marker geknüpft ist, der eine Signaleinheit umfasst, die ein quantifizierbares Signal erzeugen kann, das mit Mitteln ausgewählt aus photometrischen Techniken, spektrophotometrischen Techniken, visuellen, colorimetrischen Techniken, chemilumineszierenden Techniken, fluorometrischen Techniken und immunofluorometrischen Techniken nachweisbar ist;
(c) Erzeugen des quantifizierbaren Signals; und
(d) Nachweisen des quantifizierbaren Signals, wodurch die Polynucleotidsequenz nachgewiesen wird.

2. Verfahren nach Anspruch 1, wobei das Substrat eine Oberfläche umfasst, die selbst oder mit oder ohne Behandlung ein einzelsträngiges oder doppelsträngiges Polynucleotid daran fixieren oder immobilisieren kann und dennoch die Hybridisierung eines nicht-radioaktiv, chemisch markierten Polynucleotids an das fixiert oder immobilisierte Polynucleotid erlaubt.

3. Verfahren nach Anspruch 1 oder 2, wobei das Erzeugen des Signals das enzymatische Erzeugen eine Produkts umfasst, das visuell, colorimetrisch, über Chemilumineszenz, fluorometrisch, immunofluorometrisch, spektrophotometrisch oder photometrisch gemessen werden kann.

4. Verfahren nach Anspruch 1 oder 3, wobei das Substrat eine transparente oder transluzente Oberfläche umfasst, die so angepasst ist, dass sie den Nachweis mit Mitteln ausgewählt aus photometrischen Techniken, spektrophotometrischen Techniken, visuellen, colorimetrischen Techniken, chemilumineszierenden Techniken, fluorometrischen Techniken und immunofluorometrischen Techniken erlaubt.

5. Verfahren nach einem der Ansprüche 1 bis 4, wobei Licht durch das Substrat für die colorimetrische oder photometrische Bestimmung des Signals gesendet wird.

6. Verfahren nach einem der Ansprüche 1 bis 5, wobei das Substrat ein epoxyüberzogenes Substrat umfasst.

7. Verfahren nach einem der Ansprüche 1 bis 5, wobei das Substrat Polystyrol, Polyethylen oder Polypropylen umfasst.

8. Verfahren nach einem der Ansprüche 1 bis 7, wobei durch den Nachweis des quantifizierbaren Signals eine qualitative Bestimmung der Polynucleotidsequenz und die quantitative Bestimmung der Polynucleotidsequenz bereitgestellt wird.

9. Verfahren nach einem der Ansprüche 1 bis 8, wobei die Polynucleotidsequenz mit dem Substrat verbunden wird und das Substrat sich in einer Vorrichtung befindet.

10. Verfahren nach einem der Ansprüche 1 bis 9, wobei die Signaleinheit ein Coenzym einschließt.

11. Verfahren nach Anspruch 10, wobei das Coenzym ausgewählt ist aus der Gruppe bestehend aus Thiaminpyrophosphat, Flavinmononucleotid, Flavinadenindinucleotid, Nicotinamidadenindinucleotid, Nicotinamidadenindinucleotidphosphat, Coenzym A, Pyridoxylphosphat, Biotin, Tetrahydrofolsäure, Coenzym B₁₂, Liponsäure und Ascorbinsäure.

12. Verfahren nach einem der Ansprüche 1 bis 9, wobei die Signaleinheit einen Chelatbildner umfasst.

13. Verfahren nach einem der Ansprüche 1 bis 12, wobei die Polynucleotidsequenz an eine Vertiefung auf der Oberfläche fixiert oder immobilisiert ist, wobei die Oberfläche für den Durchgang von Licht durch diese hindurch für die photometrische oder colorimetrische Bestimmung des Signals angepasst ist.

14. Verfahren nach einem der Ansprüche 1 bis 13, weiterhin umfassend einen oder mehr Waschschritte.

15. Verfahren zur Bestimmung des quantifizierbaren Vorhandenseins eines ausgewählten genetischen Materials, wobei das Verfahren das Behandeln einer Probe umfasst, die das ausgewählte genetische Material enthält, um das genetische Material zu denaturieren, um einen Einzelstrang davon bereitzustellen, der von dem genetischen Material stammt, das Fixieren oder Immobilisieren des entstandenen Einzelstrangs des genetischen Materials an eine Glas- oder Kunststoffoberfläche, das Inkontaktbringen des so fixierten oder immobilisierten Einzelstrangs des genetischen Materials unter Hybridisierungsbedingungen mit einer nicht-radioaktiv, chemisch markierten Polynucleotidsonde, die im Wesentlichen zu den Nucleotidbestandteilen komplementär ist, die das zu bestimmende genetische Material kennzeichnen, sowie das Nachweisen einer Duplexbildung zwischen dem Einzelstrang des genetischen Materials und der nicht-radioaktiv, chemisch markierten Polynucleotidsonde durch photometrische Techniken.

16. Verfahren zur Bestimmung des quantifizierbaren Vorhandenseins eines ausgewählten genetischen Materials, wobei das Verfahren das Behandeln einer Probe umfasst, die das ausgewählte genetische Material enthält, um das genetische Material zu denaturieren, um einen Einzelstrang davon bereitzustellen, der von dem genetischen Material stammt, das Fixieren oder Immobilisieren des entstandenen Einzelstrangs des genetischen Materials an eine Glas- oder Kunststoffoberfläche, das Inkontaktbringen des so fixierten oder immobilisierten Einzelstrangs des genetischen Materials unter Hybridisierungsbedingungen mit einer nicht-radioaktiv, chemisch markierten Polynucleotidsonde, die im Wesentlichen zu den Nucleotidbestandteilen komplementär ist, die das zu bestimmende genetische Material kennzeichnen, Erzeugen eines nachweisbaren quantifizierbaren Signals durch eine enzymverbundene Immunadsorptionstesttechnik, sowie das Nachweisen einer Duplexbildung zwischen dem Einzelstrang des genetischen Materials und der nicht-radioaktiv, chemisch markierten Polynucleotidsonde durch Nachweisen des quantifizierbaren Signals.

17. Verfahren nach Anspruch 15 oder 16, wobei Licht durch die Oberfläche zur Bestimmung der Duplexbildung gesendet wird.

18. Verfahren nach Anspruch 17, wobei das einzelsträngige genetische Material an eine Vertiefung auf der Oberfläche fixiert oder immobilisiert ist, wobei die Oberfläche für den Durchgang von Licht durch diese hindurch für die photometrische oder colorimetrische Bestimmung der Duplexbildung zwischen dem einzelsträngigen genetischen Material und der Polynucleotidsonde angepasst ist.

19. Verfahren nach einem der Ansprüche 15 oder 17 bis 18, wobei die Duplexbildung zwischen dem Einzelstrang des genetischen Materials und der nicht-radioaktiv, chemisch markierten Polynucleotidsonde durch enzymverbundene Immunadsorptionstesttechniken nachgewiesen oder bestimmt wird.

20. Verfahren nach einem der Ansprüche 15 bis 19, wobei der Nachweis der Duplexbildung zwischen dem einzelsträngigen genetischen Material und der nicht-radioaktiv, chemisch markierten Polynucleotidsonde durch die Bildung eines Komplexes mit einem chemischen Marker auf der Polynucleotidsonde bestimmt wird,
wobei der Komplex ein Enzym umfasst, sowie Herausfinden des Vorhandenseins des enzymenthaltenden Komplexes, der an den chemischen Marker gebunden ist, durch Inkontaktbringen des enzymenthaltenden, an die Polynucleotidsonde gebundenen Komplexes mit einem Substrat, das bei Kontakt mit dem Enzym eine chemische Reaktion oder Farbveränderung erfährt.

21. Verfahren nach einem der Ansprüche 15 bis 18, wobei die Duplex- oder Doppelstrangbildung oder die Hybridisierung zwischen dem Einzelstrang des Materials und der nicht-radioaktiv, chemisch markierten Polynucleotidsonde **dadurch** bestimmt wird, dass vor oder nach der Duplexbildung ein Chelatbildner an dem an die Polynucleotidsonde gebundenen chemischen Marker bereitgestellt wird und das Vorhandensein des Chelatbildners durch Inkontaktbringen des Chelatbildners mit einer Substanz herausgefunden wird, die bei Kontakt mit dem Chelatbildner eine chemische Reaktion oder Farbveränderung erfährt.

22. Verfahren nach Anspruch 21, wobei die Farbveränderung photometrisch oder colorimetrisch bestimmt wird und die Menge des an die resultierende gebildete Duplex gebundenen oder verknüpften Chelatbildners anzeigt.

23. Verfahren zum Nachweisen einer Polynucleotidsequenz, umfassend:
(a) Fixieren oder Immobilisieren der Polynucleotidsequenz an ein Glas- oder Kunststoffsubstrat, so dass die Polynucleotidsequenz in einzelsträngiger Form vorliegt und an komplementäre Nucleinsäuresequenzen hybridisieren kann;
(b) Bilden einer Duplex umfassend die einzelsträngige Polynucleotidsequenz, die an eine Oligo- oder Polynucleotidsonde hybridisiert ist, wobei an die Sonde ein nicht-radioaktiver, chemischer Marker geheftet ist, der eine Signaleinheit umfasst, die ein lösliches Signal erzeugen kann;
(c) Erzeugen des löslichen Signals; und
(d) Nachweisen des erzeugten löslichen Signals, wodurch die Polynucleotidsequenz nachgewiesen wird.

24. Verfahren zum Nachweisen einer Polynucleotidsequenz, umfassend:
(a) Bilden einer Duplex, die die Polynucleotidsequenz in einzelsträngiger Form an hybridisiert eine Oligo- oder Polynucleotidsonde umfasst, wobei an die Sonde ein nicht-radioaktiver, chemischer Marker geheftet ist, der eine Signaleinheit umfasst, die ein quantifizierbares Signals erzeugen kann;
(b) Fixieren oder Immobilisieren der Duplex an ein Glas- oder Kunststoffsubstrat, so dass die Duplex ein quantifizierbares Signal erzeugen kann;
(c) Erzeugen eines quantifizierbaren Signals, das durch Mittel ausgewählt aus photometrischen Techniken, spektrophotometrischen Techniken, visuellen, colorimetrischen Techniken, chemilumineszierenden Techniken, fluorometrischen Techniken und immunofluorometrischen Techniken nachgewiesen werden kann; und
(d) Nachweisen des quantifizierbaren Signals, wodurch die Polynucleotidsequenz nachgewiesen wird.

25. Verfahren nach Anspruch 24, wobei das Erzeugen des quantifizierbaren Signals durch enzymverbundene Immunadsorptionstesttechniken erfolgt.

## Revendications

1. Méthode de détection d'une séquence polynucléotidique qui comprend :
(a) fixer ou immobiliser ladite séquence polynucléotidique à un substrat en verre ou en plastique de telle manière que la séquence polynucléotidique soit sous forme simple-brin et soit capable de s'hybrider à des séquences d'acide nucléique complémentaires ;
(b) former un duplexe comprenant ladite séquence polynucléotidique simple-brin hybridée à une sonde oligo- ou polynucléotidique, ladite sonde étant pourvue d'un marqueur chimique non-radioactif comprenant une partie de signalisation capable de générer un signal quantifiable détectable par un moyen sélectionné parmi les techniques photométriques, les techniques spectrophotométriques, visuellement, les techniques colorimétriques, les techniques de chimioluminescence, les techniques fluorométriques et les techniques immunofluorométriques ;
(c) générer ledit signal quantifiable ; et
(d) détecter ledit signal quantifiable, détectant ainsi ladite séquence polynucléotidique.

2. Méthode selon la revendication 1, dans laquelle ledit substrat comprend une surface qui est capable *per se,* ou avec ou sans traitement, de fixer ou immobiliser un polynucléotide simple-brin ou double-brin et de permettre encore l'hybridation d'un polynucléotide marqué chimiquement non-radioactif au polynucléotide fixé ou immobilisé.

3. Méthode selon la revendication 1 ou 2, dans laquelle la génération du signal comprend la génération enzymatique d'un produit qui peut être mesuré visuellement, par colorimétrie, par chimioluminescence, par fluorométrie, par immunofluorométrie, par spéctrophotométrie ou par photométrie.

4. Méthode selon la revendication 1 ou 3, dans laquelle le substrat comprend une surface transparente ou translucide adaptée pour permettre une détection par un moyen sélectionné parmi les techniques photométriques, les techniques spectrophotométriques, visuellement, les techniques colorimétriques, les techniques de chimioluminescence, les techniques fluorométriques et les techniques immunofluorométriques.

5. Méthode selon l'une quelconque des revendications 1 à 4, dans laquelle la lumière est transmise à travers le substrat pour une détermination colorimétrique ou photométrique dudit signal.

6. Méthode selon l'une quelconque des revendications 1 à 5, dans laquelle ledit substrat comprend un substrat recouvert d'époxy.

7. Méthode selon l'une quelconque des revendications 1 à 5, dans laquelle ledit substrat comprend le polystyrène, le polyéthylène, ou le polypropylène.

8. Méthode selon l'une quelconque des revendications 1 à 7, dans laquelle la détection dudit signal quantifiable fournit une détermination qualitative de la séquence polynucléotidique, et une détermination quantitative de la séquence polynucléotidique.

9. Méthode selon l'une quelconque des revendications 1 à 8, dans laquelle ladite séquence polynucléotidique est associée au dit substrat et ledit substrat est présent dans un appareil.

10. Méthode selon l'une quelconque des revendications 1 à 9, dans laquelle ladite partie de signalisation inclut un co-enzyme.

11. Méthode selon la revendication 10, dans laquelle ledit co-enzyme est sélectionné parmi le groupe consistant en une thiamine pyrophosphate, une flavine mononucléotide, une flavine adénine dinucléotide, une nicotinamide adénine dinucléotide, une nicotinamide adénine dinucléotide phosphate, le co-enzyme A, un pyridoxyl phosphate, une biotine, l'acide tétrahydrofolique, le co-enzyme B12, l'acide lipoïque et l'acide ascorbique.

12. Méthode selon l'une quelconque des revendications 1 à 9, dans laquelle ladite partie de signalisation comprend un agent chélateur.

13. Méthode selon l'une quelconque des revendications 1 à 12, dans laquelle ladite séquence polynucléotidique est fixée ou immobilisée à un puits présent sur la surface, ladite surface étant adaptée pour la transmission de lumière à travers elle pour une détermination photométrique ou colorimétrique dudit signal.

14. Méthode selon l'une quelconque des revendications 1 à 13, comprenant en outre une ou plusieurs étape de lavage.

15. Méthode pour déterminer la présence quantifiable d'un matériel génétique sélectionné, qui comprend le traitement d'un échantillon contenant ledit matériel génétique sélectionné de manière à dénaturer celui-ci pour fournir un simple-brin de celui-ci à partir du matériel génétique, la fixation ou l'immobilisation du simple-brin du matériel génétique résultant à une surface en verre ou en plastique ; la mise en contact dans des conditions d'hybridation du simple-brin du matériel génétique fixé ou immobilisé avec une sonde polynucléotidique marquée chimiquement non-radioactive substantiellement complémentaire de composants nucléotidiques caractérisant ledit matériel génétique destiné à être déterminé, et la détection de la formation de duplexe entre ledit simple-brin du matériel génétique et ladite sonde polynucléotidique marquée chimiquement non-radioactive par des techniques de photométrie.

16. Méthode pour déterminer la présence quantifiable d'un matériel génétique sélectionné, qui comprend le traitement d'un échantillon contenant ledit matériel génétique sélectionné de manière à dénaturer celui-ci pour fournir un simple-brin de celui-ci à partir du matériel génétique, la fixation ou l'immobilisation du simple-brin du matériel génétique résultant à une surface en verre ou en plastique ; la mise en contact dans des conditions d'hybridation du simple-brin du matériel génétique fixé ou immobilisé avec une sonde polynucléotidique marquée chimiquement non-radioactive substantiellement complémentaire de composants nucléotidiques caractérisant ledit matériel génétique destiné à être déterminé, la génération d'un signal quantifiable détectable par technique d'essai par immunosorbant lié à une enzyme, et la détection de la formation de duplexe entre ledit simple-brin du matériel génétique et ladite sonde polynucléotidique marquée chimiquement non-radioactive par détection dudit signal quantitatif.

17. Méthode selon la revendication 15 ou 16, dans laquelle la lumière est transmise à travers ladite surface pour déterminer ladite formation de duplexe.

18. Méthode selon la revendication 17, dans laquelle ledit matériel génétique simple-brin est fixé ou immobilisé à un puits présent dans ladite surface, ladite surface étant adaptée pour la transmission de lumière à travers elle pour une détermination photométrique ou colorimétrique de la formation de duplexe entre ledit matériel génétique simple-brin et ladite sonde polynucléotidique.

19. Méthode selon l'une quelconque des revendications 15 ou 17 à 18, dans laquelle ladite formation de duplexe entre ledit simple brin du matériel génétique et ladite sonde polynucléotidique marquée chimiquement non-radioactive est détectée ou déterminée par des techniques d'essai par immunosorbant lié à une enzyme.

20. Méthode selon l'une quelconque des revendications 15 à 19, dans laquelle la détection de la formation de duplexe entre ledit simple-brin du matériel génétique et ladite sonde polynucléotidique marquée chimiquement non-radioactive est déterminée par la formation d'un complexe avec un marqueur chimique présent sur ladite sonde polynycléotidique, ledit complexe comprenant une enzyme, et la mise en évidence dudit complexe contenant une enzyme attaché au dit marqueur chimique en mettant ledit complexe contenant une enzyme attachée à ladite sonde polynucléotidique en contact avec un substrat qui subit une réaction chimique ou un changement de couleur lorsqu'il est en contact avec ladite enyme.

21. Méthode selon l'une quelconque des revendications 15 à 18, dans laquelle la formation de duplexe ou de double-brin entre ledit simple-brin du matériel génétique et ladite sonde polynucléotidique marquée chimiquement non-radioactive est déterminée en fournissant, avant ou après la formation du duplexe, un agent chélateur au dit marqueur chimique attaché à ladite sonde polynucléotidique, et en mettant en évidence la présence dudit agent chélateur par mise en contact dudit agent chélateur avec une substance qui subit une réaction chimique ou un changement de couleur lorsqu'elle est en contact avec ledit agent chélateur.

22. Méthode selon la revendication 21, dans laquelle le changement de couleur est déterminé par photométrie ou colorimétrie et est indicatif de la quantité d'agent chélateur fixé ou attaché au duplexe formé résultant.

23. Méthode pour détecter une séquence polynucléotidique qui comprend :
(a) fixer ou immobiliser ladite séquence polynucléotidique à un substrat en verre ou en plastique de telle manière que la séquence polynucléotidique soit sous forme simple-brin et soit capable de s'hybrider à des séquences d'acide nucléique complémentaires ;
(b) former un duplexe comprenant ladite séquence polynucléotidique simple-brin hybridée à une sonde oligo- ou polynucléotidique, ladite sonde étant pourvue d'un marqueur chimique non-radioactif comprenant une partie de signalisation capable de générer un signal soluble ;
(c) générer ledit signal soluble : et
(d) détecter le signal soluble généré, détectant ainsi ladite séquence polynucléotidique.

24. Méthode pour détecter une séquence polynucléotidique qui comprend :
(a) former un duplexe comprenant la séquence polynucléotidique sous forme simple-brin hybridée à une sonde oligo- ou polynucléotidique, ladite sonde étant pourvue d'un marqueur chimique non-radioactif comprenant une partie de signalisation capable de générer un signal quantifiable ;
(b) fixer ou immobiliser ledit duplexe à un substrat en verre ou en plastique de telle manière que le duplexe soit capable de générer un signal quantifiable ;
(c) générer un signal quantifiable détectable par un moyen sélectionné parmi les techniques photométriques, les techniques spectrophotométriques, visuellement, les techniques colorimétriques, les techniques de chimioluminescence, les techniques fluorométriques et les techniques immunofluorométriques ; et
(d) détecter ledit signal quantifiable, détectant ainsi ladite séquence polynucléotidique.

25. Méthode selon la revendication 24, dans laquelle ladite génération dudit signal quantifiable est faite par des techniques d'essai par immunosorbant lié à une enzyme.
